# EUROPÄISCHE PATENTSCHRIFT

(11) **EP 4 008 904 B1**
(45) Veröffentlichungstag und Bekanntmachung des Hinweises auf die Patenterteilung: **25.12.2024**
(21) Anmeldenummer: 21212330.1
(22) Anmeldetag: 03.12.2021
(51) Int. Cl.: F04C 2/22, F04C 13/00, F04C 15/06, A61M 60/196, A61M 60/441, A61M 60/835, A61M 60/894, A61M 60/896, A61M 60/258

(54) **PUMPE ZUR HERZUNTERSTÜTZUNG**
PUMP USED FOR CARDIAC SUPPORT
POMPE D'ASSISTANCE CARDIAQUE

(30) Priorität: 03.12.2020 DE 102020132226
(43) Veröffentlichungstag der Anmeldung: 08.06.2022
(73) Patentinhaber: RAP-P Meditec UG, 25881 Tating (DE)
(72) Erfinder: Horn, Franz-Harro, 25881 Tating (DE)
(74) Vertreter: Schied, Sebastian

(56) Entgegenhaltungen:
- DE-U1- 202016 000 016
- US-A1- 2019 167 872
- US-B2- 6 576 010

## Beschreibung

Die Erfindung betrifft eine Pumpe zur Herzunterstützung. Die Pumpe soll einen Pulsschlag nachstellen und den Blutfluss sehr schonend unterstützen.

Weltweit wird intensiv an den Möglichkeiten einer mechanischen Unterstützung eines unter Herzinsuffizienz leidenden Menschen gearbeitet und sogar an einem teilweise technischen Herzersatz.

Systeme, die den Pulsschlag nachstellen sollten, weisen beispielsweise Lösungen mit gummiartigen Pumpkammern oder mebranartigen Folien auf, die der Werkstoffalterung und damit einem Verschleiß unterliegen. Darum setzt man heute zum Beispiel einfache Zentrifugalpumpsysteme ein, die sich weitgehend durch ihre Einfachheit bewähren jedoch nur einen pulsfreien und damit stetigen gleichmäßigen Blutfluss generieren, der gegebenenfalls oder nur ansatzweise verschiedene Drücke wellenartig auf Grund variabler Drehzahlerhöhung erzeugen können, um damit dem Einfluss des Pulses näher zu kommen. Bedacht muss dabei werden, dass hohe Drehzahlen einer derartigen Lösung einen durchaus negativen Einfluss auf die Belastbarkeit und damit auf die Transportfunktion des Blutes haben. Allerdings kann bei derartigen technischen Konstruktionen allein mit metallischen wie keramischen nicht dem Verschleiß unterliegenden Materialien gearbeitet werden, was auch so zu deren heutigen Einsatz z. B. bei den Zentrifugalsystemlösungen führte.

Bei den Zentrifugalpumpsystemen kommt es jedoch auf Grund hoher Drehzahl schon im Ansaugbereich zu einer radialen Verwirbelung um den Ansaugvektor mit anschließender weiterer starker Radialbeschleunigung durch den schnell drehenden Antriebsrotor im eigentlichen Pumpenkörper.

Aus dem hydraulischen Pumpenbau sind Drehkolbenpumpen mit zwei ineinander verkämmt laufenden Drehkolben oder einem Drehkolben und einer Dichtklappe bekannt. Bekannt sind auch Flügelpumpen, die eine alternierende Drehschwenkbewegung eines Flügels mit Ventilklappen im Flügel und im Pumpengehäuse für den Einlass und den Auslass aufweisen. Problemtisch bei den an sich bekannten Pumpen ist, dass sich strömungsfreie Toträume bilden können, so dass bei der Verwendung als Blutpumpe die Gefahr der Bildung von Blutgerinseln und/oder Plaque besteht.

Bekannt ist beispielsweise aus der DE 10 2004 005 468 B4 eine Rotationskolbenmaschine, einsetzbar als Pumpe für Flüssigkeiten, welche einen Arbeitsrotor und einem Dichtrotor aufweist, wobei die zu fördernden Fluide über eine zentrale Hohlwelle zugeführt werden. Nachteilig ist die damit verbundene große Bauform.

Weiterhin ist aus der DE6927594U eine Drehkolbenpumpe mit einem Drehkolben und einer Dichtklappe bekannt.

Weiterhin ist aus der DE202016000016U1 eine Drehkolbenpumpe mit einem in einem Pumpengehäuse rotierbaren Drehkolben und zumindest zwei Außenläufern bekannt.

Die US 2019/167872 A1 offenbart ein künstliches Herz mit einer Pumpe, wobei die Pumpe ein Gehäuse mit einem im Wesentlichen kugelförmigen Hohlraum und vier Gefäßanschlüsse umfasst. Eine drehbare Scheibe ist im Gehäuse angebracht und so befestigt, dass sie sich um eine feste Achse dreht, während zwei oszillierende Paletten so angebracht sind, dass sie sich um eine bewegliche Achse drehen, die in einer Ebene senkrecht zur festen Achse beweglich ist, wobei die Paletten miteinander verbunden und auf beiden Seiten der drehbaren Scheibe (11) diametral gegenüberliegend angeordnet sind, um zwei Pumpeinheiten zu bilden, die jeweils zwei Kammern variabler Größe umfassen, die mit einem Gefäßanschluss in Fluidverbindung stehen. Im Betrieb erfolgt eine oszillierende Bewegung jeder oszillierenden Palette relativ zu der drehbaren Scheibe, um gleichzeitig zwei Ansaughübe und zwei Ausstoßhübe zu erzeugen, so dass Blut in eine Kammer jeder Pumpeinheit und Blut aus der anderen Kammer jeder Pumpeinheit durch die entsprechenden Gefäßanschlüsse zu pumpen. Die Antriebseinheit der Pumpe erzeugt ein rotierendes Magnetfeld innerhalb des Pumpengehäuses.

Aufgabe der Erfindung ist es, eine Drehkolbenpumpe bzw. Drehflügelpumpe zu schaffen, welche membranfrei ist und in der Lage ist, einen Puls während der Pumparbeit nachzustellen Dabei soll eine schonende Förderung des jeweiligen Fluides, beispielsweise Blut erreicht werden.

Es hat sich gezeigt, dass Drehkolbenpumpen bzw. Drehflügelpumpen mit einem speziellem Drehkolben mit zumindest zwei Flügeln und zumindest zwei Dichtklappen als Herzpumpe gut geeignet sind.

Die erfindungsgemäße Drehkolbenpumpe bzw. Drehflügelpumpe ist in der Lage einen Puls während ihrer Pumparbeit nachzustellen. Die besonderen Grundbedingungen eines ein menschliches Herz ersetzenden Gerätes lassen sich berücksichtigen.

Mit der Erfindung wird im angegebenen Anwendungsfall erreicht, dass eine Drehkolbenpumpe bzw. Drehflügelpumpe, umfassend ein Pumpengehäuse mit einer im Wesentlichen zylindrischen Pumpenkammer und einem Drehkolben als Rotor mit zumindest zwei in Umfangsrichtung gegenüberliegend oder gleichmäßig verteilt angeordneten Flügeln, ein Rotorachsrohr und zumindest einer Dichtklappe geschaffen wird, wobei zumindest zwei in Umfangsrichtung gegenüberliegend oder gleichmäßig verteilt angeordnete Dichtklappen vorhanden sind, und wobei die zumindest zwei Dichtklappen drehbar oder schwenkbar sind und wobei in dem Rotorachsrohr sich jeweils von den gegenüberliegenden axialen Enden erstreckend und voneinander getrennt axial ein Einlaufkanal zu zumindest zwei Einlassöffnungen in die Pumpenkammer und ein Auslaufkanal axial von zu zumindest zwei Auslassöffnungen aus der Pumpenkammer vorhanden ist, wobei jeweils eine der Einlassöffnungen in Rotationsrichtung nach und jeweils eine der Auslassöffnungen in Rotationsrichtung vor jeweils einem der Flügel im Rotorachsrohr angeordnet ist. Hierbei schließt drehbar oder schwenkbar auch verschiebbar oder verdrehbar mit ein.

In Bezug auf die Rotationsrichtung bilden sich vor dem jeweiligen Flügel eine Druckseite oder Ablaufseite bzw. ein entsprechender Bereich und in Rotationsrichtung nach dem jeweiligen Flügel eine Sogseite oder Zulaufseite bzw. ein entsprechender Bereich.

Weiterhin umfasst die Erfindung ein Verfahren zum Betreiben einer Drehkolbenpumpe bzw. Drehflügelpumpe, bei welchem in einer im Wesentlichen zylindrischen Pumpenkammer eines Pumpengehäuses durch die Drehbewegung eines Drehkolbens als Rotor mit einem Rotorachsrohr und mit daran zumindest zwei in Umfangsrichtung gegenüberliegend oder gleichmäßig verteilt angeordneten Flügeln ein Fluid in jeweils eine Auslassöffnung in dem Rotorachsrohr in Rotationsrichtung vor dem jeweiligen Flügel gepresst und zugleich das Fluid durch eine Einlassöffnung in dem Rotorachsrohr in Rotationsrichtung nach dem jeweiligen Flügel angesaugt wird, wobei eine Trennung der Bereiche des Pressens und des Ansaugens jeweils mittels einer Dichtklappe erfolgt und die jeweilige Dichtklappe sich für den Druck oder Ablauf und den Sog oder Zulauf in einer geschlossenen Stellung befindet und nach dem Druck oder Ablauf und dem Sog oder Zulauf für das Passieren des jeweiligen Flügels die jeweilige Dichtklappe an den radial äußeren Bereich der Pumpenkammer geschwenkt oder um das radial äußeren Ende des jeweiligen Flügels gedreht wird und nach dem Passieren die jeweilige Dichtklappe wieder in eine geschlossene Stellung an das Rotorachsrohr herangeführt bzw. bewegt wird und damit die Pumpenkammer wieder in die in Umfangrichtung abgegrenzten Bereiche für einen Druck oder Ablauf und Sog oder Zulauf schließt, womit die Blutförderung bzw. der Druck oder Ablauf und der Sog oder Zulauf ein weiteres Mal getaktet erneut erfolgt. Durch das Passieren des jeweiligen Flügels durch die notwendig ausweichende jeweilige Dichtklappe ist der Blutfluss solange vollständig unterbrochen, bis der Flügel den Bereich der Dichtklappe passiert hat bzw. durchlaufen hat und die Dichtklappe sich danach wieder schließt bzw. sich schließend bewegt.

Erfindungsgemäß ist in der im Wesentlichen zylindrischen Pumpenkammer des Pumpengehäuses, beispielsweise mit einem vorderem und hinterem Gehäusedeckel ein Rotor zumindest zwei Flügeln angeordnet, der mittels seiner Rotation Blut bewegt.

Vorteilhaft wird in der hier vorgestellten Pumpe das Fluid weitgehend nur laminar bewegt, teilweise bewusst beschleunigt und auch in der Pumpe während des Pumpens ähnlich wie im natürlichen Herzen gepresst und beschleunigt ausgeworfen.

Ein das Blut traumatisierendes rotierendes extremes Verwirbeln wie in den bekannten Zentrifugalpumpen , VAD-Pumpen, mit i. M. 5000 U/min des Rotors erfolgt nicht. Jedoch wird ein nahe dem natürlichen Eindrehen des Blutes aus einem gesunden Herzen in das Adersystem sehr wohl erreicht. Dies ist ein in allen Naturvorgängen zu beobachtendes Auftreten besonders bei Fluiden, Mäandern der Flüsse, wie auch den Bewegungen von Gasen. Im gesunden Körper wird dieser Vorgang eines leichten Verwirbelns eingegeben womit das Blut leicht in alle Richtungen wirbelnd am Bilden von Thromben und Plaque gehindert wird.

Die Pumpe dreht mit 35 U/min und mit einer halben Umdrehung werden 2 x 35 cm³ = 70 cm³ je Puls gefördert. Der Rotor fördert das Blut durch eine Drehbewegung über einen Bereich von ca. 140° Drehwinkel je Puls innerhalb einer halben Umdrehung bzw. innerhalb von 180° Drehwinkel in nur ca. 0,24 Sekunden. Eine halbe Umdrehung entspricht einem Puls entsprechend 70 Pulsen im Mittel je Minute. Generell übergibt diese Pumpenbauart mittels ihrer moderaten Drehung eine solche an das zu fördernde Medium, was für den Fall der Anatomie der Blutbewegung von hoher Wichtigkeit ist und somit zur Verhinderung von Thromben- und Plaquebildungen beiträgt. Vorteilhaft oder vorzugsweise weist dafür ergänzend der Auslaufkanal oder das Rotorachsrohr für den Auslauf oder das Ausgangsrohr einen gewunden oder schraubenförmigen Verlauf oder Innenquerschnitt auf.

Das Fluid beschreibt seinen Fluss durch klare vorgegebene Führungsquerschnitte ohne scharfe Kanten etc.. Mit beispielsweise 35 U/min bzw. 70 Pulsen je Minute kann das Blut keinen signifikanten Schaden nehmen. Nur beim relativ langsamen Passieren des Flügels unter der Dichtklappe kommt es zu einer allseitigen aber sehr wohl gewollten aber moderaten Verwirbelung des Blutes, um Verdickung oder Plaquebildung zu vermeiden. Die Bewegungen der Komponenten sind jedoch vergleichsweise ruhige Bewegungen sehr glatter Oberflächen im Medium Blut, die an dieser Stelle bewusst nach allen Seiten die Klappe als auch den Flügel frei umspült wie spülend so vorgesehen sind.

Die erfindungsgemäße Drehkolbenpumpe bzw. Drehflügelpumpe weist zumindest zwei Dichtklappen auf. Auf Grund der Unterbrechung der Förderleistung durch die vor bezeichnete und zum Passieren des Flügels sich öffnende Dichtklappe aber sonst die zeitliche und/oder räumliche Begrenzung der Pumpenkammer mit den Pumpenkammerabschnitten darstellend, ergibt sich eine Taktung beziehungsweise Pulsung des Fluids als Fördergut.

Dabei läuft es für diesen medizinischen Zweck zur Nutzung als pulsende Blutförderpumpe, also einer teilweisen wie einseitigen Herzunterstützung oder gar als vollständiger Herzersatz und damit der Arbeit für beide Herzkammern hinaus. Das Adersystem wird durch den von der Pumpe ausgehenden Puls einer Dehnung als auch vollkommenen Entlastung unterzogen ähnlich der Arbeit des natürlichen Herzens. Damit wird nicht nur das Ader- sondern auch das Ventrikelsystem pulsierend elastisch erhalten.

Vorteilhaft ist für den jeweils voneinander getrennten Auslaufkanal und Einlaufkanal, die sich jeweils von den gegenüberliegenden axialen Enden des Rotorachsrohrs erstrecken, und für die Zuströmung und die Abströmung des Fluids das Rotorachsrohr axial in zwei Abschnitte geteilt ist, wobei quer zur axialen Erstreckung des Rotorachsrohrs eine strömungsoptimierte, beispielsweise schrägen, auch gerundeten wie gebogenen Kanaltrennung vorgesehen ist und damit der Einlaufkanal einem Zuströmkanal von einem axialen Ende in das Rotorachsrohr und der Auslaufkanal einem Abströmkanal vom gegenüberliegenden axialen Ende aus das Rotorachsrohr bilden, wobei das Fluid durch den Einlaufkanal als Zuströmkanal über die Einlassöffnungen in die Pumpenkammer gelangt und das Fluid die Pumpenkammer durch die Auslassöffnungen in den Auslaufkanal als Abströmkanal wieder verlässt. Damit liegen die Anschlüsse für die Zuströmung und die Abströmung des Fluids in einer Linie.

Die erfindungsgemäße Drehkolbenpumpe bzw. Drehflügelpumpe stellt eine Unterstützung des menschlichen Herzens oder erlaubt die teilweise Auswechselung eines Herzens gegen einen künstlichen Ersatz. Dabei stellt die erfindungsgemäße Drehkolbenpumpe einen grundsätzlichen Pulsschlag ähnlich dem eines gesunden Herzens beispielsweise nahe 70 Pulsen/min bereit, indem über einen Antrieb ein je nach Patient bestimmtes bzw. ausgelegtes Fördervolumen von beispielsweise ca. 70 cm³ pro Umdrehung und damit um beispielsweise 4.900 cm³/min Blut elektronisch gesteuert gepumpt wird. Dabei kann je nach Leistungserfordernis die Frischblutversorgung an den jeweiligen Körper angepasst auch variabel pulsend in den Blutkreislauf erfolgen.

Die einfache Konstruktion umfasst an sich drei bewegte sich drehende oder teildrehende Teile, umfassend Rotor mit Flügel und die beiden Dichtklappen, und ein im Wesentlichen kreisrundes zylindrisches Pumpengehäuse aus metallischen oder anderen nicht alternden Materialien.

Die erfindungsgemäße Drehkolbenpumpe bzw. Drehflügelpumpe ermöglicht es, einen grundsätzlichen Pulsschlag ähnlich dem eines gesunden Herzens im Normalpulsmodus mit ca. 70 Pulsen je Minute, jedoch auch mit deutlich gesteigerter oder geminderter Fördermenge, eingestellt durch die Drehzahl, bereitzustellen.

Die aktive Ausbringung des Blutes aus der Drehkolbenpumpe bzw. Drehflügelpumpe folgt beispielsweise direkt der Vorgabe aus dem Sinusknoten, die messtechnisch übernommen digital an die Steuerungseinheit geleitet und als elektrischer Befehl weitergegeben ist.

Hierbei lässt sich die Fördermenge sowohl für kleine als auch für große Herzen sowie für größere oder geringere Fördervolumina anpassen, indem die Größe der Pumpenkammer in Bezug auf die Länge oder auch ihrem Durchmesser dem gewünschten Fördervolumen des speziellen Patienten angepasst wird und damit einfach skalierbar verschiedene Leistungsgrößen für den Einsatz zur Verfügung stehen.

So lassen sich auch die Drehzahl der Pumpe auf der Basis des zu messenden Sauerstoffbedarfs oder weiterer physiologischer Kenndaten anpassen, damit der Puls ähnlich dem eines menschlichen Herzens bei erhöhten Leistungsanstrengungen oder Leistungserfordernis in der Lage ist, kurzzeitig als auch dauerhaft sich anzupassen, sofern diese physiologischen Informationen messtechnisch zur Verfügung gestellt werden können und beispielsweise mittels einer Software eine höhere Drehzahl dem Antrieb beispielsweise dem Drehfeld eines Elektromotors software- wie hardwaremäßig vorgegeben wird.

Den konstruktiven Aufbau vereinfachend ist eine einfache Rotorlagerung vorgesehen, wenn der Rotor beispielsweise zwei Flügel in Bezug auf eine Umdrehung bzw. den Vollkreis bei 0° und bei 180° vorsieht und damit pro halber Umdrehung das gewünschte Fördervolumen gepumpt bzw. bewegt werden kann und die Anordnung der Flügel des Rotors die Pumpenkammer in beispielsweise zwei Förderhälften teilt, so das zwei getrennte Förderbereiche über jeweils eine halbe Umdrehung das vorbestimmte Volumen bereitstellen und das Rotorachsrohr in der axialen Erstreckung mittig eine Kanaltrennung aufweist. Bei dieser Lösung mit zwei gegenüberliegenden Förderabschnitten hebt sich der auftretende Druck, anders als bei einem Rotor mit nur einem Flügel der auftretende einseitige Druck, auf, wodurch der Rotor schwimmend mit seinen zwei Flügeln geführt ist und damit eine nur einfache Lagerungslösung vorgesehen werden kann.

Das Rotorachsrohr des Rotors lässt sich im Pumpengehäuse beispielsweise an zwei Gehäusehälften oder an zwei Gehäusedeckeln an einem Gehäuse oder auf einer konzentrisch angeordneten Zentrier- oder Führungs-Achse oder -Welle oder auf andere geeignete Weise lagern.

Das Rotorachsrohr kann dabei auf der Oberfläche der jeweiligen Gehäusekomponente oder in Vertiefung gelagert laufen oder in entsprechende Öffnungen des Einlaufes oder Auslaufes hineinragen und dort gelagert sein.

Im Pumpengehäuse bilden sich durch die Flügel ein erster und ein zweiter Förderbereich über einen Bereich jeweils einer halben Umdrehung des Rotors. Das Volumen beträgt beispielsweise zweimal ca. 35 cm³ je halbe Umdrehung, so dass 70 cm³ je Puls gepumpt werden können.

Zudem sind gegebenenfalls an den Flügeln Magneten und am Pumpengehäuse an der Gehäusewand und/oder den Gehäusedeckeln, beispielsweise an den beiden in axialer Richtung vorhandenen Gehäuseflächen in Umfangrichtung umlaufend Elektromagneten vorhanden. Mittels der Anordnung Magneten bzw. der Elektromagneten und deren Ansteuerung lässt sich der Rotor auch rein auf elektromagnetisch-elektronischer Vermessung zentrieren, indem eine entsprechende Ansteuerung durch eine damit verbundenen Mehr- oder Minderbeaufschlagung der Elektromagnete erfolgt. Weiterhin lassen sich zusätzlich in Abhängigkeit steigender oder fallender notwendiger Blutmengen variierenden Rotationsgeschwindigkeiten des Rotors erwirken.

Kommt ein Rotor mit nur einem Flügel zum Einsatz, wäre dieser zu lagern, da durch die Druckarbeit ein nicht unerheblicher Seitendruck auf den Rotor ausgeübt wird und damit ohne Lagerung die Funktion eines ungestörten Rotorumlaufes nicht gewährt wäre.

Weiterhin kann das Pumpengehäuse mehrteilig aufgebaut sein. So kann das Pumpengehäuse beispielsweise durch zwei Gehäusehälften oder mit zwei Gehäusedeckeln an einem Gehäuse oder anderweitig mehrteilig gebildet bzw. zusammengesetzt sein.

Vorteilhaft kann auf einen Dichtrotor verzichtet werden, wodurch sich die Baugröße des Kunstherzens deutlich verringern kann. Statt des Dichtrotors kommen die Dichtklappen zum Einsatz, welche vorzugsweise gebogen oder sichelförmig oder omega-förmig sind und die Begrenzung der Pumpenkammer und des jeweiligen Pumpenkammerabschnittes bilden. Damit wird eine kleinere Baugröße erreicht. Die Dichtklappen lassen sich über beispielsweise einen mechanischen oder elektro-mechanischen oder magnetischen Antrieb am oder im Pumpengehäuse beispielsweise in oder an dem Gehäusedeckel oder den Gehäusedeckeln synchron ansteuern und bewegen, um den Flügel passieren zu lassen und danach wieder zu schließen. Die Bewegung der Dichtklappe ist abhängig von der Winkelgeschwindigkeit des Rotors bzw. vom Drehwinkel der Flügel und damit des Rotors.

Vorzugsweise lassen sich zwei der Drehkolbenpumpen kaskadieren oder kombiniert anordnen, so dass mit mehreren Drehkolbenpumpen beispielsweise parallel unterschiedliche Fluide oder das gleiche Fluid gepumpt werden können. So ist eine Kombination als Doppelpumpe möglich. Als Herzersatz oder Herzunterstützung wird Frischblut aus den Lungengefäßen übernommen und axial in die Aorta geleitet. Die parallele Pumpe übernimmt das Altblut der Hohlvenen und leitet es in die Lungenarterien. Diese Funktionen lassen sich auch mit nur einer gemeinsamen Rotorachs umsetzen. Damit können zwei Pumpen mit einer gemeinsamen Rotorachse bzw. Rotorwelle oder jeweils getrennt oder versetzt mit jeweils einer eigenen Rotorachse bzw. Rotorwelle synchron ihre Arbeit verrichten.

Ein weiterer erfindungsgemäßer Aspekt erlaubt es, dass zwei Pumpen gekoppelt mit einer gemeinsamen Rotorachse bzw. Rotorwelle versehen sind. Eine der beiden Pumpen übernimmt mit deren Rotorachsrohr das Blut der Lungenvenen und leitet es durch die Pumpenkammer wieder in das andere Ende des Rotorachsrohrs und von dort in die Aorta . Gleichermaßen wird das Blut der Hohlvenen in das Rotorachsrohr der anderen Pumpe eingeleitet. Nach dem Passieren der zweiten oder anderen Pumpenkammer wird es ebenfalls wieder in das andere Ende des Rotorachsrohrs geleitet und in die Lungenarterien übergeben. Damit lassen sich zwei Pumpen auf einer Rotorachse bzw. Rotorwelle gemeinsam und synchron aber getrennt ihre Arbeit verrichten. Der Austritt des Blutes aus dem Ausgangsrohr in das jeweilige Blutgefäß ist derart angelegt, dass er mit dem Puls eindeutig kongruent harmoniert.

Die Bezeichnung Rotationsgeschwindigkeit bzw. Winkelgeschwindigkeit des Rotors bzw. dessen Flügel werden als synonym verstanden.

Die Drehkolbenpumpe kann wegen der Form des Rotors und der Flügel auch als Drehflügelpumpe bezeichnet werden bzw. stellt eine Drehflügelpumpe dar. Insofern lassen sich die beiden Bezeichnungen synonym verwenden.

Als Fluid werden beliebige Flüssigkeiten so auch Blut oder andere förderbare Körperflüssigkeiten verstanden.

Vorteilhafte Ausgestaltungen der Drehkolbenpumpe und des Verfahrens sind in den Unteransprüchen dargestellt.

Indem die Flügel punktsymmetrisch zur Rotationsache des Rotorachsrohrs sind, lassen sich vorteilhaft strömungsoptimierte sowie an das Fluid angepasste Flügel vorsehen.

Indem die Kontur der Flügel in radialer Richtung einen einfach oder mehrfach gekrümmten Verlauf aufweisen, lässt sich die Verdrängung des Fluids optimieren. Insbesondere im Bereich vor und an den Dichtklappen lässt sich durch eine entsprechende Formung die Verdrängung der Fluidmenge optimieren, um damit die Baugröße der Pumpe so gering als möglich zu gestalten. Wegen der anatomischen Besonderheiten bzw. wegen des begrenzten Raumes beispielsweise im Brustraum eines Menschen, ist eine geringe Baugröße zu bevorzugen.

Vorteilhaft weist die Kontur der zumindest zwei Dichtklappen in radialer Richtung des Pumpengehäuses einen gekrümmten oder kreisbogenförmigen Verlauf auf. In Bezug auf die Krafteinwirkung durch Druck oder Ablauf und Sog oder Zulauf beim Pumpen wird dadurch die Kräfteverteilung optimiert. Weiterhin lässt sich die Verdrängung des Fluids insbesondere im Zusammenwirken mit dem jeweiligen Flügel des Rotors weiter optimieren. Die jeweiligen Dichtklappen sind somit flächig bzw. teilzylindermantelförmig über den Bereich bzw. Umfangsabschnitt des Kreisbogens.

Indem der in radialer Richtung gekrümmte Verlauf der Kontur der Flügel und der gekrümmte Verlauf der Kontur der zumindest zwei Dichtklappen zueinander ähnlich oder angepasst oder kongruent sind, lässt sich die Verdrängung des Fluids im Zusammenwirken mit dem jeweiligen Flügel des Rotors weiter optimieren.

Indem die zumindest zwei Dichtklappen an dem jeweils zur radial äußeren Gehäusewand der Pumpenkammer weisenden Ende eine annähernd tangential geradlinige Verlängerung oder eine Verlängerung mit einem oder dem Eintritts- bzw. Austrittsradius entsprechend des Pumpenkammeraußendurchmessers der Gehäusewand der Pumpenkammer bzw. einem oder dem Pumpenkammeraußendurchmesser aufweisen, wird erreicht, dass der jeweilige Flügel noch Pumparbeiten leisten kann, während er sich bereits im Bereich der Dichtklappe bzw. Omega-Klappen befindet, ohne dass diese sich bereits öffnet oder öffnen muss. Hierdurch wird die Zeitspanne bzw. der Drehwinkel minimiert, in der die Pumpe keine Pumparbeit leistet oder leisten kann. Ein weiterer Vorteil besteht darin, dass das Gesamtvolumen der Pumpe bei gleichbleibender Pumpleistung reduziert werden kann. Weiterhin wird das Vorbeilaufen bzw. Einlaufen des jeweiligen Flügels in den Bereich der jeweiligen Dichtklappe begünstigt. Zudem wird die Dichtwirkung an der radial äußeren Wand des Pumpengehäuses sowie am Rotorachsrohr verbessert.

Indem die zumindest zwei Dichtklappen im oder am Pumpengehäuse über Speichen oder Stege oder Stifte oder Kugeln an einem Drehpunkt oder Schwenkpunkt oder in Schienen oder in oder auf Führungen gleitbar oder drehbar oder schwenkbar gelagert oder angeordnet sind, wobei das Pumpengehäuse im Bereich oder Bewegungsbereich der zumindest zwei Dichtklappen zumindest bereichsweise radial und/oder axial zumindest einen Freiraum aufweist, also das Pumpengehäuse in diesem Bereich geweitet ist, lassen sich die Dichtklappen optimal lagern und für das Passieren des jeweiligen Flügels bewegen oder ausweichen. Zudem wird durch den beim Öffnen der jeweiligen Dichtklappe entstehenden Freiraum seitlich, oberhalb wie unter der Dichtklappe eine Verwirbelung des Fluides im Bereich der Lagerung und Führung und damit im Bereich der Weitung des Pumpengehäuses erzwungen und begünstigt.

Indem die zumindest zwei Dichtklappen konzentrisch oder exzentrisch drehbar oder schwenkbar oder verdrehbar sind, wobei die zumindest zwei Dichtklappen in einer geschlossenen Position radial und axial am Pumpengehäuse anliegen und in geöffneter Position vom Pumpengehäuse beabstandet sind, lässt sich die Lage bzw. der Abstand der Dichtklappen zum Pumpengehäuse vergrößern, wodurch eine Umströmung und Verwirbelung des Fluides im Grenzbereich zwischen Dichtklappe und Pumpengehäuses begünstigt wird. Insbesondere beim Umgang bzw. dem Pumpen von Blut sind Vorkehrungen zu treffen, dass gefährliche Blutgerinnsel vermieden werden. Hierzu ist es wichtig, dass es keinen Bereich gibt, in den das Blut langsam oder nicht fließt.

Bevorzugt weisen die zumindest zwei Dichtklappen in radialer Richtung eine Sichel-förmigen oder Omega-förmigen Verlauf auf. Die Dichtklappe lässt sich damit auch als Omega-Klappe bezeichnen. Sie weist damit einen im Zusammenwirken mit den Flügeln des Rotors optimierten Verlauf auf.

Die Begriffe Dichtklappe und Omega-Klappe werden vereinfachend auch synonym verwendet, so dass unter einer Omega-Klappe eine Dichtklappe verstanden wird und mit einer Dichtklappe auch eine Omega-Klappe gemeint ist.

Vorzugsweise weist die Konstruktion der Omega-Klappe, ein im Wesentlichen gleichschenkliges Omega an seinem Schenkel einen Flügel auf der, der am Pumpenkammeraußenradius dem Umfang folgt und den anfänglichen Ein- und Durchlauf des jeweiligen Flügels des Rotors zur weitgehenden Ausräumung einer restlichen Blutmenge bis in die Omega-Klappe hinein ermöglicht.

Die jeweilige Omega-Klappe ist um einen exzentrischen Drehpunkt drehend bzw. schwenkbar oder verdrehbar gelagert. Mit dem beiderseitigen axialen Außenrändern ist die jeweilige Omega-Klappe am jeweiligen axialen Pumpengehäuseabschluss beispielsweise den Gehäusedeckeln entsprechend verdrehbar angelegt. Die jeweilige Omega-Klappe bewegt sich zum Öffnen zunächst in radialer Richtung so, dass der jeweilige Flügel des Rotors in einer synchronisierten Drehung zwischen Rotor und der Omega-Klappe frei passieren kann, wobei zusätzlich bereits mit dem Einsetzen der Öffnung der Dichtklappe sich die radial dichtenden Anlagebögen der Gehäusewandung mit der Dichtklappe vom Pumpengehäuse entfernen, womit ein aktives Umströmen an den Außenrändern der nun allseits frei liegenden Dichtklappe gewährleistet ist, beispielsweise oberhalb und unterhalb sowie seitlich dieser Freiräume zum Pumpengehäuse. Dass Pumpengehäuse ist also im Bereich oder Bewegungsbereich der zumindest zwei Dichtklappen zumindest bereichsweise radial und/oder axial geweitet.

Vorteilhaft ist im Bereich der jeweiligen Dichtklappe bzw. Omega-Klappe radial außen wie jeweils axial ein Abstand zwischen der jeweiligen Dichtklappe bzw. Omega-Klappe und dem passierenden Flügel vorhanden.

Dieser Abstand entspricht annähernd dem oder bevorzugt dem Freiraum bzw. der Ausweitung zwischen der jeweiligen Dichtklappe bzw. Omega-Klappe und der im Bereich der jeweiligen Dichtklappe bzw. Omega-Klappe geweiteten radialen Gehäusewand bzw. dem in diesem Bereich geweiteten axialen Gehäusedeckel.

Damit lässt sich mit der verwirbelnden Blutmenge durch die allseitig freie Bewegung für hinreichende Unruhe und Verwirbelung sorgen, dass mit einer Bildung von verdickendem Blut nicht zu rechnen ist.

Auf Grund dieser besonderen Gestaltung sowie auch Lagerung der Dichtklappe werden die in ihr auftretenden Kraftvektoren, Druck und Sog, mit sich selber zu Null ausgeglichen, somit werden die Druck- und die Sogkräfte während der Förderphase im Inneren und Äußeren der Omega-Klappe weitgehend gegenseitig aufgehoben. Damit wird die Positionierung der Omega-Klappe deutlich vereinfacht. Weiterhin ist auch der Energiebedarf zum Halten der Omega-Klappe sehr gering, womit nur eine Führungseinrichtung zum Öffnen und Schließen, die auch die Position in der geöffneten oder geschlossenen Stellung der Omega-Klappe bestimmt und sichert, vorgehalten.

Vorteilhaft hat im Innern der Herzpumpe ein vollständiger Dichtigkeitsgrad zwischen der Druckseite und Sogseite nicht notwendig vollkommen zu sein. Seitliche Dichtungen an den Flügeln sind somit nur bei Bedarf notwendig.

Vorteilhaft ist ein geringer Spalt, beispielsweise von 0,08 mm bis 0,3 mm zwischen den Flügeln des Rotors und der radialen Gehäusewand sowie der axialen Gehäusedeckel des Pumpengehäuses vorgesehen, um möglichst eine geringe Bluttraumatisierung zu erreichen. Damit ist weitgehend ein in Drehung befindliches aber dem Takt des menschlichen Herzens angeglichenes sowie schonendes Pumpen vorbestimmt.

Im geschlossen Zustand der jeweiligen Dichtklappe liegt diese an der radialen Gehäusewand sowie der axialen Gehäusedeckel sowie am Rotorachsrohr an.

Es kann jedoch zumindest an den axialen Gehäusedeckeln sowie am Rotorachsrohr ein geringer Spalt vorhanden sein.

Indem das Pumpengehäuse zumindest im radial äußeren Bereich über den Umfang und/oder über die axiale Erstreckung und/oder im Bereich der Dichtklappen gleichmäßig und/oder unregelmäßig verteilt Elektromagnete aufweist, lassen sich einerseits die Lage und Bewegung der Flügel und damit des Rotors erfassen und anderseits auch ein einfacher und berührungsloser Antrieb der Flügel und damit des Rotor realisieren. Zudem kann auch einerseits die Lage und Bewegung der Dichtklappen überwacht und gesteuert werden.

Indem zumindest am radial äußeren Ende der Flügel zumindest ein Magnet vorhanden ist, lassen sich die Rotationsgeschwindigkeiten des Rotors und damit der Flügel und/oder die Stellung der Flügel in Umfangsrichtung erfassen. Insbesondere kann mittels des oder der Magneten am Flügel ein Antrieb unter Einsatz der Elektromagneten am Pumpengehäuse realisiert werden, welcher die Flügel und damit den Rotor präzise und bedarfsgerecht antreibt.

Indem an den zumindest zwei Dichtklappen ein, zwei oder mehr Magnete vorhanden sind, lassen sich diese unter Einsatz der Elektromagneten von außerhalb oder innerhalb des Pumpengehäuses oder durch die Magneten an den Flügeln von innen berührungslos ansteuern. Um elektromagnetische Felder ungehindert zum Antrieb nutzen zu können, ist eine entsprechende Wahl beispielsweise von metallischen Werkstoffen einerseits bzw. keramischen oder faserverstärkten Kunststoffen andererseits notwendig.

Alternativ kann die Ansteuerung und Bedienung der Dichtklappen mechanisch erfolgen, beispielsweise mittels einer Rastung und/oder mittels Federkraft. Dabei kann der jeweilige Flügel unterstützend eingreifen. Der Flügel kann das Öffnen und/oder das Schließen der Dichtklappe auslösen oder unterstützen.

Indem die Elektromagnete einzeln und/oder als Gruppe ansteuerbar sind, kann die Ansteuerung, beispielsweise die Beschleunigung, Geschwindigkeit und Verzögerung der Dichtklappen und/oder der Flügel und damit des Rotors begünstigt werden. Zudem lässt sich auch eine Lageüberwachung der Dichtklappen und/oder der Flügel des Rotors verbessern. Mittels der Elektromagneten lässt sich ein variables Drehfeld bereitstellen.

Indem die zumindest zwei Dichtklappen jeweils zumindest eine Dichtung aufweisen, wird der Fluidfluss kontrolliert über die Einlassöffnungen und Auslassöffnungen geleitet. Diese können bevorzugt im Bereich zumindest eines der jeweiligen radialen Enden der Dichtklappen vorhanden sein.

Indem die Flügel radial im Bereich des Pumpengehäuses in Rotationsrichtung einen Überstand aufweisen lassen sich die jeweiligen Dichtklappen in eine ausreichende oder vollständige geöffnete Stellung bewegen, damit die Flügel des Rotors problemlos passieren können. Somit kann die Ansteuerung der jeweiligen Dichtklappen vereinfacht werden.

Diese Pumpenbauart stellt sich als Zwangspumpe dar, wodurch zwangsweise das Fluid eingesogen wird. Zum Abbau oder zum Ausgleichen ungünstigerer Druckverhältnisse, welche sich auch negativ auf die Peripherie auswirken können, ist jeweils ein Überströmventil als unterdruckgesteuerter Bypass in der jeweiligen Dichtklappe zwischen dem Abschnitt Druck oder Ablauf, also in Rotationsrichtung vor der jeweiligen Dichtklappe, und dem Abschnitt Sog oder Zulauf, in Rotationsrichtung nach der jeweiligen Dichtklappe, vorhanden

Indem ein Überströmventil oder unterdruckgesteuerter Bypass zwischen Druck oder Ablauf, also dem Abschnitt der in Rotationsrichtung dem Flügel vorauseilt, und Sog oder Zulauf, also dem Abschnitt der in Rotationsrichtung dem Flügel nacheilt, aufweisen, lassen sich alternativ oder zusätzlich ungünstigere Druckverhältnisse ausgleichen oder ein gezielte Überströmen bewirken.

Ein solcher Bypass kann aber auch an oder im Gehäuse oder an anderer Stelle vorgesehen sein.

Weiterhin sind alternativ oder zusätzlich in den Flügeln und/oder am Pumpengehäuse im Bereich der Dichtklappen von der Druckseite zur Sogseite ein Überströmventil oder unterdruckgesteuerter Bypass vorhanden, um ungünstige Druckverhältnisse auszugleichen oder ein gezielte Überströmen bewirken zu können.

Die erfindungsgemäße Pumpe ist eine Zwangspumpe. Bei der Konfiguration einer derartigen Pumpe, kommt es zu einem zwangsweisen Sog, der bei zu wenig Blutvorrat in der bzw. in den Herzkammern zu einer Implosion der Kammern führen kann. Damit tritt ein kritischer Zustand ein. Vorteilhaft ist ein unterdruckgesteuerter Bypass vorgesehen, der an oder in der Pumpe verhindert, dass bei Auftreten eines vordefinierten Unterdruckes mechanisch oder elektrotechnisch gesteuert ein Ventil den Bypass zwischen dem Druckbereich und der Sogseite frei gibt und gerade gefördertes Blut auf direktem Wege auf die Sogseite zurückleitet und ein weiteres Absinken des Unterdruckes auf der Sogseite verhindert. Damit wird ähnlich dem gesunden Herzen dann zwar das bekannte Schwindelgefühl auf Grund nicht genügender sauerstoffreicher Blutmenge im Körper und vor allem im Kopf eintreten. Dies wird jedoch den Körper zum Innehalten zwingen jedoch keine große Gefahr entstehen lassen, bis der Kreislauf sich im Körper durch Innehalten und nach Erkennen mittels der Steuerung eine elektronische Steueranweisung an die Rotationsgeschwindigkeit der Pumpe zur Normalisierung des Pulsverhaltens und damit des Körperwohlfühlens führt.

Weiterführend zu diesem obigen Thema, kann gefolgert werden. Wenn eine Zwangspumpe die zu fördernde Blutmenge an der unteren Spitze der linken Herzkammer weitgehend entnimmt, also den dem gesunden Herzen entsprechenden Inhalt von in Mittel 70 cm³, so ist in dieser Herzkammer nahezu kein Blut mehr vorhanden, dass durch die Herzmuskelkontraktion auf natürlichem Wege durch die Aortaklappe in die Aorta gepumpt werden kann. Damit ist nur eine Restmenge Blut vorhanden, die das oberhalb der Aortaklappe in der dort sehr weiten Aorta befindliche Blut auf natürlichem Wege nur wenig oder gar nicht bewegen kann und es somit dort zu Stauungen kommen wird. Bekannt ist, dass stauendes bzw. stehendes Blut dazu neigt, zu verdicken oder gar zu klumpen und es zu Thrombosen führen kann.

Um dieses zu unterbinden, wird die bekannte OP-Technik des Einsatzes der an sich bekannten VAD-Pumpen an der unteren Herzkammer bezüglich der hier vorgestellten Pumpe schwerlich zu einem befriedigenden Erfolg führen. Dies bedeutet, dass die erfindungsgemäße Drehflügelpumpe beispielsweise besser oberhalb des Herzmuskels wie der Aortaklappe und zum Übergang in die Aorta platziert werden kann. Damit wäre der natürliche Blutfluss wieder hergestellt. Die Segelklappen in die weite Aorta würden beispielsweise in das Eingangsrohr der Pumpe ragen und das Ausgangsrohr würde vom anderen Ende der getrennten Aorta darüber umfasst.

Indem die Drehbewegung bzw. die Winkelgeschwindigkeit des Drehkolbens, also des Rotors und damit der Flügel mit je nach Winkelstellung der Flügel unterschiedlicher Rotationsgeschwindigkeit erfolgt, werden unterschiedliche Drücke und Strömungsgeschwindigkeiten erreicht. Dazu gehört auch, dass der Rotor für eine Zeitspanne zum Stillstand kommen oder angehalten werden kann, um danach beschleunigt bzw. wieder bewegt die Pumparbeit aufzunehmen.

Vorteilhaft wird die Drehbewegung bzw. die Winkelgeschwindigkeit des Drehkolbens verringert, wenn sich der jeweilige Flügel im Bereich der jeweiligen Dichtklappe befindet. Insbesondere im Bereich der Dichtklappen und beim Passieren der Dichtklappen ist es von Vorteil, die Rotationsgeschwindigkeit zu reduzieren. Ebenso ist es je ja nach Trägheit und Ansteuerung der Dichtklappen von Vorteil, die Rotationsgeschwindigkeit zu reduzieren. Es hat sich gezeigt, dass mit den unterschiedlichen Rotationsgeschwindigkeiten und der damit verbundenen Fördermengen, eine nahezu humanvergleichende Pulsung des Fluids oder des Bluts erreicht wird.

Somit werden beispielsweise nach dem ein Verzögern der Geschwindigkeit der Flügel im Bereich und beim Passieren der Dichtklappen erfolgte und die Flügel bzw. der Rotor nachdem sie die Dichtklappen passiert haben wieder beschleunigt, wodurch die Pumparbeit wieder aufgenommen und gezielt ein Puls oder Pulsschlag mit einer herzähnlichen Pulskurve erzeugt wird.

Alternativ zur Verlangsamung kann auch ein zeitweiser Stillstand der Flügel im Bereich der Dichtklappen vorgesehen werden.

Befinden sich die Flügel im Bereich und beim Passieren der Dichtklappen verringert sich die Pumparbeit oder kommt zum Erliegen. Diese Zeitspanne, in dem sich die Flügel im Bereich der Dichtklappen befinden oder diese passieren, entspricht etwa dem 0,7 bis 0,75-fachen der Zeit für eine halbe Umdrehung, also dem Weg den ein Flügel von Dichtklappe zu Dichtklappe zurücklegt. Wie bekannt, erfolgt die Pulsung des Herzens im Mittel mit 70 Schlägen je Minute, also 0,84 Sekunden je Herzarbeit, aufgeteilt in ca. 0,6 Sekunden zum Auffüllen der jeweiligen Herzkammer aus dem jeweiligen Vorhof sowie ca. 0,24 Sekunden zum Austreiben aus der jeweiligen Herzkammer in die Aorta bzw. Lungenaterie durch Kontraktion der Herzmuskel. In diesen erstgenannten 0,6 Sekunden bewegt sich bei der erfindungsgemäßen Pumpe die Dichtklappe zum Öffnen ca. 0,1 Sekunden und verweilt für ca. 0,4 Sekunden und bewegt sich zum Schließen in weiteren ca. 0,1 Sekunden durch ein Rückdrehen in die Ausgangs- bzw. Dichtlage. Für das Öffnen und Schließen dreht sich die Dichtklappe im Pumpengehäuse jeweils mit ca. 110° Drehwinkel.

Diese Arbeit kann diese technische Pumpe nahezu gleichermaßen bedienen, indem die Pumpe mit dem Rotor und somit mit den beiden Flügeln die Pumpenkammer bzw. die Pumpenkammerabschnitte binnen 0,24 Sekunden überwindet und das Fluid bzw. Blut mittels der bekannten Schlauchprothese an die Aorta und/oder auch an die Lunge abgibt. Dann passieren die beiden Flügel die Dichtklappe bzw. Omega-Klappe binnen 0,6 Sekunden mit einer oben beschriebenen an sich aktiven das Blut allseits bewegenden Förderunterbrechung. Dies gilt gleichermaßen auch für eine beiderseitige herzunterstützende Maßnahme wie bei einem Herzersatz.

So lassen sich dann die Drehbewegung der Flügel bzw. des Rotors wieder anlaufend die Pumparbeit beschleunigt aufnehmen, ein dem menschlichen Herzschlag täuschend ähnlichen Puls nachzustellen.

Die Drehbewegung des Rotors und/oder die Stellung der Dichtklappen und/oder alle weiteren messtechnischen Daten als Basis für den Betrieb der Pumpe lassen sich erfassen und der Betrieb der Pumpe gesteuert gestalten. Dazu werden vorzugsweise auch die auftretenden Bewegungs-, Anlauf- bzw. der Beschleunigungsmomente berücksichtigt.

Indem die Einstellung der Drehbewegung des Drehkolbens und/oder Stellung der Dichtklappen mittels der Ansteuerung bevorzugt von Elektromagneten erfolgt, wird ein präzises Zusammenwirken der Dichtklappen und der Flügel des Rotors begünstigt. Indem im Auslaufkanal eine Segelklappe vorgesehen ist, lässt sich ein Rückstau verhindern, sofern beim Durchlauf des/der Flügel unter der Dichtklappe eine ungewollt größere Menge Blut zurückstauen sollte.

Vorteilhaft erfolgt eine Früherkennung des Stromimpulses des Sinusknotens und/oder des AV-Knotens, wobei dieser Impuls kurzfristig verstärkt und über eine Computerschnittstelle an die Antriebseinheit, beispielsweise einer Elektromotoreinheit oder die Magnetsteuerung geleitet wird, um damit eine weitgehende Synchronisation des Kunstherzens mit dem menschlichen Takt basierend auf den physiologischen Zuständen zu gewährleisten.

Vorzugsweise verrichtet die Herzpumpe für Patienten der Nordhalbkugel des Planeten Erde die Pumparbeit mit einer Linksdrehung, während Sie für Patienten der südlichen Halbkugel rechtsdrehend arbeitet.

Die Drehkolbenpumpe kann als Herzpumpe zur Unterstützung des menschlichen Herzens oder als beidseitiger Herzersatz verwendet werden.

Mehrere Ausführungsbeispiele der Erfindung sind in den Zeichnungen dargestellt und werden im Folgenden näher beschrieben. Es zeigen:
Fig. 1 eine Drehkolbenpumpe bzw. Drehflügelpumpe als Schnittdarstellung in Draufsicht, wobei die Flügel des Rotors in radialer Richtung gerade sind und die Dichtklappen eine nur gering gekrümmten Verlauf der Kontur aufweisen,
Fig. 2 bis 4 jeweils eine Drehkolbenpumpe bzw. Drehflügelpumpe als Schnittdarstellung in Draufsicht in unterschiedlichen Lagen und Stellungen des Rotors und der Dichtklappen, wobei die Flügel des Rotors in radialer Richtung gekrümmt sind und die Dichtklappen sichelförmig oder omega-förmig gekrümmt sind,
Fig. 5 eine Drehkolbenpumpe bzw. Drehflügelpumpe als teilweise Detailschnittdarstellung der Figur 2 in seitlicher Schnittdarstellung,
Fig. 6 eine Drehkolbenpumpe bzw. Drehflügelpumpe in seitlicher Schnittdarstellung am Ende des Förderns, kurz vor dem Öffnen der Dichtklappen,
Fig. 7 eine Detailschnittdarstellung des Flügels und der Dichtklappe aus Figur 3
Fig. 8 eine Detailschnittdarstellung des Flügels und der Dichtklappe
Fig. 9 und 10, jeweils eine Drehkolbenpumpe bzw. Drehflügelpumpe in seitlicher Schnittdarstellung in unterschiedlichen Lagen und Stellungen des Rotors und der Dichtklappen, wobei die Flügel des Rotors in radialer Richtung gekrümmt sind und die Dichtklappen sichelförmig oder omega-förmig gekrümmt sind,
Fig. 11 eine Detailschnittdarstellung des Flügels und der Dichtklappe mit vergrößerter Darstellung der Freiräume bei Öffnen der Klappe und
Fig. 12 eine seitliche Schnittdarstellung der Figur 11.

Das Grundprinzip für eine Herzunterstützung mit der erfindungsgemäßen Drehkolbenpumpe bzw. Drehflügelpumpe stellt sich beispielsweise wie folgt dar. Das frische Blut wird beispielsweise aus der Lunge kommend einem zentralen Eingang 9 beispielsweise in einem Gehäusedeckel 9 an einem axialen Ende des Pumpengehäuses 2 und einem im Wesentlichen gleich großen oder sich verengenden Einlaufkanal 11 in einem Rotorachsrohr 18 eines drehenden Rotors 1 der Drehkolbenpumpe bzw. Drehflügelpumpe zugeführt. Das Blut gelangt weiter durch zwei Einlassöffnungen 6 im Rotorachsrohr 18 in die Pumpenkammer 8 bzw. den jeweiligen Pumpenkammerabschnitten 8a. Durch den Pumpvorgang gelangt das Blut aus der Pumpenkammer 8 bzw. aus den jeweiligen Pumpenkammerabschnitten 8a über zwei Auslassöffnungen 7 im Rotorachsrohr 18 in den Auslaufkanal 12 der zu dem Ausgang 10 beispielsweise in einem weiteren Gehäusedeckel 10 aus dem Pumpengehäuse 2 führt und wird dem entsprechenden peripheren Blutgefäß zugeführt.

Das Rotorachsrohr 18 des Rotors 1 erstreckt sich vom Eingang 9 an einem axialen Ende zu einem Ausgang 10 an einem gegenüberliegenden Ende des Rotorachsrohrs 18. Im Rotorachsrohr 18 ist eine gerade oder gerundete Kanaltrennung 19 zwischen Eingang 9 und Ausgang 10 bzw. zwischen dem Einlaufkanal 11 und dem Auslaufkanal 12 vorhanden, die eine Strömung begünstigend zu den Einlassöffnungen 6 und zu den Auslassöffnungen 7 schräg verläuft, damit den Einlaufkanal 11 von dem Auslaufkanal 12 im Rotorachsrohr 18 trennt. Damit wird das Rotorachsrohr 18 quer zur axialen Erstreckung strömungstechnisch geteilt.

Der Rotor 1 generiert je nach elektronisch-elektrischer Ansteuerung durch entsprechende Umdrehungen nahe 70 Pulse je Minute. Die Flügel 3 streichen an den Gehäusewänden 2 der Pumpenkammer 8 entlang. Zu den Gehäusewänden 2 der Pumpenkammer 8 zählen die radial äußere Wand des Pumpengehäuses 2 der Pumpenkammer 8 sowie die Wandungen der axialen Begrenzung der Pumpenkammer 8, beispielsweise die Innenwände der Gehäusedeckel 9, 10 des Pumpengehäuses 2. Die Flügel 3 grenzen eine Druckseite 7 zu einer Sogseite 6 ab. Da das Fördervolumen der Pumpenkammer 8 mit den jeweiligen Pumpenkammerabschnitten 8a zu zweimal ca. 35 cm³ durch den Raum zwischen Rotorachsrohr 18 und den Gehäusewänden 2 der Pumpenkammer 8 definiert ist und damit beispielsweise im Mittel 70 cm³ Fördervolumen je halbe Umdrehung, tritt bei Drehung des Rotors 1 bzw. der jeweiligen Flügel 3 in Richtung der jeweiligen Dichtklappe 4a, 4b vor dem jeweiligen Flügel 3 ein Druck 7 und hinter dem jeweiligen Flügel 3 ein Sog 6 auf. Die Dichtklappen 4a, 4b beispielsweise als Omega-Klappen 4b unterteilen die Pumpenkammer 8 in zwei Pumpenkammerabschnitte 8a und so wird das Fördergut je Pumpenkammerabschnitt 8a vor dem jeweiligen Flügel 3 durch eine Öffnung als Auslassöffnung 7 in das Rotorachsrohr 18 gedrückt und hinter dem jeweiligen Flügel 3 wird es durch die Öffnung als Einlassöffnung 6 in den jeweiligen Pumpenkammerabschnitt 8a der Pumpenkammer 8 eingesogen. Damit ergibt sich für eine derartige Lösung eine Drehzahl des Rotors 1 im Mittel bei 35 U/min.

Die Verbindung zum Adersystem wie zum Beispiel zur Aorta wird mit einer Gefäßprothese überbrückt, wie sie an sich aus dem Stand der Technik bekannt sind.

Als Dichtklappen 4b kommen vorteilhaft die Omega-Klappen 4b in Betracht, an zumindest einem radialen Ende der Kreisbogenabschnitte bzw. der Teilzylinderwandung eine Verlängerung 36 mit einem Eintritts- bzw. Austrittsradius 23 entsprechend des Pumpenkammeraußendurchmessers 23 aufweisen, um die Drehbewegung des Flügels 3 zu begünstigen und beim Einfahren weiterhin teilweise noch das jeweilige Fluid fördern zu können.

Weiterhin halten sich die Druckverhältnisse in diesem System begrenzt, sodass die Omega-Klappe 4b mechanisch oder elektromagnetisch gehalten werden kann, solange die Pumpe Medium fördert und somit auf die jeweilige Dichtklappe 4a, 4b zugleich eine Druckkraft und eine Sogkraft einwirkt. Als vorherrschende Drücke kommen die für jeweiligen Gefäße und Organe auskömmlichen Drücke in Betracht.

Die Omega-Klappe 4b kann axial und/oder radial angesteuert. Hinzu kommt eine Führung und Lagerung im oder am axialen Gehäusedeckel 9, 10 des Pumpengehäuses 2.

Die Omega-Klappe 4b kann seitlich mit einer der vielen mechanischen Lösungsmöglichkeiten in der Gehäusewand 2 gesteuert in eine Ausweitung 29 oder einen Freiraum 29 des Pumpengehäuses 2 zum Öffnen eingefahren werden.

Eine Schließung kann gesteuert oder automatisiert erzwungen erfolgen. Beispielsweise kann dazu eine im Gehäusedeckel 9 und 10 auf dem Rotorachsrohr 2 angebrachte Nockenführung, eine winkelabhängige Zahnradführung oder ähnliches vorgesehen sein, welche die Omega-Klappe 4b in Abhängigkeit des Drehwinkels des Rotors 1 und damit der Flügel 3 zum Öffnen wie Schließen bewegt.

Die Dichtklappe 4a, 4b lässt sich je nach Auslegung und bei Bedarf gegen den Förderdruck arretieren, um dann zum Durchlauf des Flügels 3 auszuweichen. Das kann ebenfalls mechanisch als auch elektromagnetisch oder ähnlich gesteuert geschehen.

Die Dichtklappe 4b als Omega-Klappe 4b ist derart geformt oder gestaltet, dass sie die an ihr auftretenden Druckkräfte und auch die Sogkräfte durch ihre omega-förmige bzw. kreisbogenförmige Ausführung mit einem Kreisbogen vom mehr 180°, weitgehend mit sich gegenseitig ausgleichen bzw. sich zu Null eliminieren.

Da im Innern der Herzpumpe ein vollständiger Dichtigkeitsgrad zwischen der Druckseite 7 und Sogseite 6 nicht notwendig ist, kann ein geringer Spalt zwischen den Flügeln 3 des Rotors 1 und der Dichtklappe 4a, 4b ebenfalls gegen die Gehäusewand 2 des Pumpengehäuses 2 vorgesehen sein, wodurch eine geringe Bluttraumatisierung erreicht werden kann und ein in Drehung befindliches und dem Takt des menschlichen Herzens angeglichenes und schonendes Pumpen vorbestimmt wird.

Beim Durchlaufen des Flügels 3 unter oder entlang der dafür sich öffnenden bzw. geöffneten oder ausweichenden bzw. ausgewichenen Omega-Klappe 4b fällt die Pumpleistung mit einer zeitlich zu definierenden Schwankung auf Null wobei durch eine fortgesetzte, jedoch verlangsamte Drehbewegung des Rotors 1 und der Flügel 3 eine starke Verwirbelung des Blutes erreicht werden soll. Das Fördern beginnt erst wieder mit Schließen der Dichtklappen 4a, 4b, wofür der jeweilige Flügel 3 den jeweiligen Bewegungsbereich bzw. Schwenkbereich der jeweiligen Dichtklappen 4a, 4b verlassen hat.

Dieser Vorgang der Förderunterbrechung dauert je nach Auslegung der einzelnen Durchmesser, nämlich des Pumpenkammerinnendurchmessers 24 bzw. des Rotorachsrohrdurchmessers 24 und des Pumpenkammeraußendurchmesser 23, und beispielsweise bei einem Flügel und einer Nockenauslegung zur Steuerung der Omega-Klappe 4b 0,11 - 0,18 Sekunden über einem Bereich des Drehwinkel von 35° bis 50° und einer Rotationsgeschwindigkeit von 70 U/min bei gleichmäßiger Rotation eines Rotors mit einem Flügel 3. Eine Pulsarbeit in Nachahmung zum menschlichen Herzen kann damit grob annähernd ermöglicht werden.

Eine darüber hinaus gehende genauere Angleichung des Pumpvorganges an das Verhalten des natürlichen Herzens kann mit dieser Pumpe wie folgt erreicht werden.

Es kann in diesem relativ langsam drehenden System im Mittel von 70 U/min unter Verwendung eines Flügels 3 oder bevorzugt mit 35 U /min unter Verwendung von zwei Flügeln 3 ein Elektroantrieb getaktet den Rotor 1 beschleunigen oder verzögern. Durch die Taktfrequenz sowie ihrer Charakteristik lassen sich der Antrieb und damit die Pumpe und deren Pumparbeit individuell noch näher an den menschlichen Puls heranführen. Die Drehzahl ist bei einem Flügel 3 mit annähernd 70 U/min für einen Normalpuls gering. Im der bevorzugten Ausführung mit 2 Flügeln 3 ist die Drehzahl dann mit 35 U/min halbiert und damit noch präziser steuerbar, da durch die doppelte und parallel Pumparbeit innerhalb einer Umdrehung ebenfalls ein Puls von 70 und das geforderte Fördervolumen Fördervolumen aus zwei Förderabschnitten zu je 35 cm³ = 70 cm³ erreicht wird.

Eine halbierte Rotationsgeschwindigkeit des Rotors 1 ergibt sich daraus, dass der Rotor 1 zwei radial gegenüber angeordneten Flügel 3 aufweist und in der Pumpenkammer 8 ebenfalls zwei radial gegenüber angeordneten Omega-Klappen 4b angeordnet sind, die mit sich gegenseitig aufhebenden Druckverhältnissen in entsprechender Bewegung sind. Bei dieser Variante erliegt die Blutförderung über ca. 30 bis 50° von 180° Drehung, je nach den gewählten Radien, also dem Pumpenkammeraußendurchmesser 23 und dem Pumpenkammerinnendurchmesser 24 bzw. Rotorachsrohrdurchmesser 24. Ca. 28 % des Zeitumfanges eines natürlichen Pulsschlags geschieht mit Beschleunigung zum Fördern über ca. 130° bis 165° einer 180° Drehung im Pumpenkammerabschnitt 8a zwischen den Omega-Klappe 4b. Im Bereich der bzw. unter der jeweiligen Omega-Klappe 4b verlangsamt der Rotor 1 mit seinen Flügeln 3 beim bzw. für das Durchlaufen unter dieser auf ca. 72 % des Zeitumfanges eines natürlichen Pulsschlags. Mit dem erneuten Beschleunigen des Rotors 1 nach der jeweiligen Omega-Klappe 4b über das ca. 0,28-fache der Förderzeit bzw. ca. 28 % des Zeitumfanges zu einem Pulsschlag wird wiederholt ein voller Pumpstoß eingeleitet und ausgestoßen, wie dies ähnlich dem Pulsen des Naturherzens erfolgt.

Die ca. 72 % des natürlichen Pulsschlags bzw. Herzrhythmus dienen der Sammlung wie der Füllung der natürlichen Herzkammer/n was hier in der Pumpe mittels Verlangsamung zum Durchlauf des jeweiligen Flügels 3 unter der jeweiligen Omega-Klappe 4b gezielt begleitet wird und danach die Pumpe der Herzkammer das Blut auf ihrer Sogseite 6 zeitgleich mit dem Pulsschlag entzieht. Somit lässt sich die Pumparbeit mit dem Takt des natürlichen Herzens synchronisieren.

Die motorische Leistung bzw. der motorische Antrieb kann mit einem elektrischen Getriebemotor sichergestellt werden. Besser ist ein direkter elektrischer Antrieb, der am Einlauf 11 wie Auslauf 12 der Gehäusedeckel 9, 10 oder direkt am äußeren Umfang der Gehäusewand 2 des Pumpengehäuse 2 zum Rotor 1 integriert werden kann.

Am radialen Außenrand des/der Flügel 3 können Magnete 17a als Permanentmagneten ihren Platz finden, die einem umlaufenden elektrischen Feld am Gehäusedeckel 9, 10 oder an der radial äußeren Wandung des Pumpengehäuses 2 folgen, wenn dort Elektromagneten 16a verbaut sind und diese entsprechend angesteuert werden.

Die einzelnen Fließgeschwindigkeiten des Blutes in der Pumpe werden durch die Wahl der jeweiligen Durchflussquerschnitte insbesondere der Einlassöffnung 6, der Auslassöffnung 7 optimierend vorbestimmt, um in der Pumpe zusätzlich für genügend Verwirbelung durch eine örtliche Beschleunigung zu sorgen.

Für einen vollständigen Pumpersatz des menschlichen Herzens bietet sich diese Pumpe ebenfalls an. Dazu ist eine Tandemlösung realisierbar, die als parallele Doppelpumpe wie die oben beschriebene Einzelpumpe arbeitet. Eine Pumpe übernimmt das Frischblut der Lungenhälften und die zweite Pumpe das rückkehrende Altblut zur Übergabe an die Lunge.

Naheliegend dazu besteht die Lösung, bei der ein Rotor 1 für beide Förderrichtungen auf der einen Seite das Frischblut aufnimmt und direkt vor der Dichtklappe 4 durch die Gehäusewand 2 oder die Gehäusedeckel 9, 10 nach außen leitend das Blut der Aorta zuführt. Das Altblut wird an dem gegenüber liegenden Rohrende eingeführt und ebenfalls seitlich aus dem Pumpengehäuse 2 dann der Lunge zugeleitet oder umgekehrt. Das Rotorachsrohr 18 ist zwischen den beiden Rohr- beziehungsweise Pumpenhälften durch eine Trennwand 30 verschlossen. Damit wird nur eine motorische Lösung benötigt statt zweier.

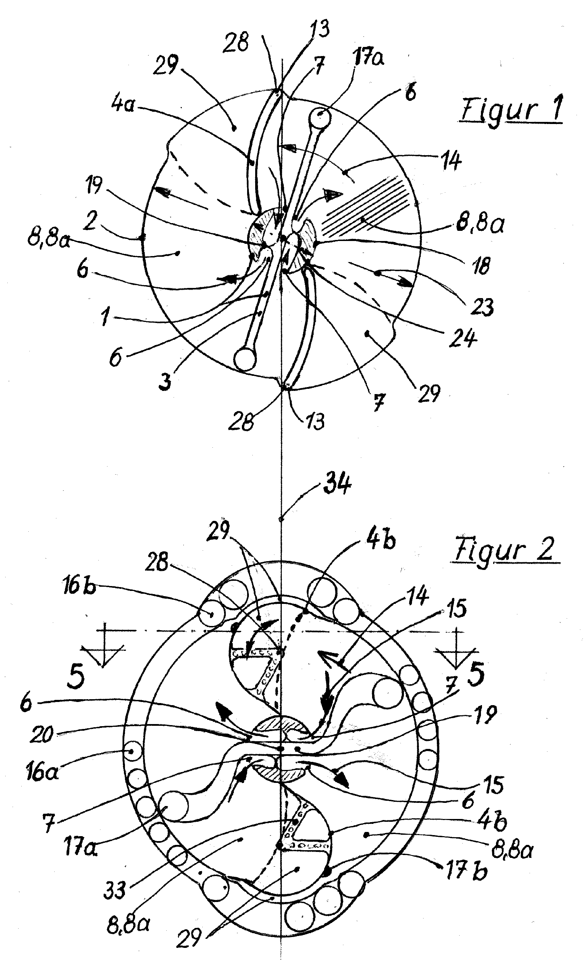

Als konkretes Ausführungsbeispiel zeigt die Figuren 1 bis 4 in Schnittdarstellung eine Drehkolbenpumpe bzw. Drehflügelpumpe mit einem Pumpengehäuse 2. Die Pumpenkammer 8 ist im Wesentlichen zylindrisch. Konzentrisch ist ein Drehkolben als Rotor 1 mit zwei in Umfangsrichtung gegenüberliegenden Flügeln 3 angeordnet. Der Rotor 1 mit den Flügeln 3 umfasst das Rotorachsrohr 18 oder mit anderen Worten bilden Rotorachsrohr 18 und der Rotor 1 mit den Flügeln 3 eine konstruktive Einheit.

In den Figuren 1 bis 4 sind im Rotorachsrohr 18 wie oben beschrieben jeweils von den gegenüberliegenden axialen Enden erstreckend und voneinander getrennt axial ein Einlaufkanal (nicht dargestellt) zu zumindest zwei Einlassöffnungen 6 in die Pumpenkammer 8 und ein Auslaufkanal (nicht dargestellt) von zu zumindest zwei Auslassöffnungen 7 aus der Pumpenkammer 8 vorhanden. Dabei ist jeweils eine der Einlassöffnungen 6 in Rotationsrichtung 14 nach und jeweils eine der Auslassöffnungen 7 in Rotationsrichtung 14 vor jeweils einem der Flügel 3 im Rotorachsrohr 18 angeordnet ist. Dementsprechend bildet sich in Bezug auf die Rotationsrichtung 14 vor dem jeweiligen Flügel 3 eine Druckseite 7 oder Ablaufseite 7 und in Bezug auf die Rotationsrichtung 14 nach dem jeweiligen Flügel 3 eine Sogseite 6 oder Zulaufseite 6.

Die Dichtklappe 4b und die Omega-Klappe 4b können als synonym verstanden werden, so dass bei alleiniger Nennung der Dichtklappe 4b auch die Omega-Klappe 4b mit umfasst ist.

Der Einlaufkanal (nicht dargestellt) und der Auslaufkanal (nicht dargestellt) sind mittels einer Kanaltrennung 19, welche das Rotorachsrohr 18 quer zur axialen Erstreckung in zwei Abschnitte aufteilt, getrennt.

Weiterhin sind in Umfangsrichtung gegenüberliegend verteilt zwei Dichtklappen 4a, 4b vorhanden sind. Die Dichtklappen 4a, 4b weisen die Dichtklappen 4a, 4b in radialer Richtung einen in der Kontur gekrümmten oder kreisbogenförmigen Verlauf auf.

Der zum Pumpengehäuse 8 radiale Verlauf der Kontur der Dichtklappen 4a gemäß Figur1 ist nur gering gekrümmt. Insbesondere die in den Figuren 2 bis 4 dargestellten Dichtklappen 4b weisen als sogenannte Omega-Klappen 4b radial zum Pumpengehäuse 8 einen in der Kontur kreisbogenförmigen bzw. teilzylindermantelförmig Verlauf auf, wobei der Kreisbogen bzw. der Teilzylindermantel über 180 Grad ausgebildet ist und die jeweiligen Enden der Omega-Klappen 4b die Verlängerung 36 mit dem Eintritts- bzw. Austrittsradius 23 entsprechend des Pumpenkammeraußendurchmessers 23 der Gehäusewand der 2 Pumpenkammer 8 aufweisen.

Zudem sind Dichtklappen 4a, 4b jeweils drehbar oder schwenkbar oder verdrehbar oder verschiebbar ausgeführt.

Die Dichtklappen 4a, 4b sind gemäß der Figuren 1 bis 4 dafür im oder am Pumpengehäuse 2 über Speichen 33 oder Stifte 13 an einem Drehpunkt 28 oder Schwenkpunkt 28 drehbar oder schwenkbar gelagert, wobei das Pumpengehäuse 2 im Bereich oder Bewegungsbereich der zwei Dichtklappen 4a, 4b bereichsweise radial und axial zumindest einen Freiraum 29 aufweist. Die radial äußere und axiale Gehäusewand 2 im Bereich oder Bewegungsbereich der zwei Dichtklappen 4a, 4b weist damit eine Ausweitung 29 auf, in welche die jeweilige Dichtklappen 4a, 4b zumindest teilweise hineindrehen oder hindurchdrehen oder hineinschwenken kann, wodurch der jeweilige Flügel 3 mit einer maximalen radialen Länge entlang oder an der jeweiligen Dichtklappe 4a, 4b vorbeistreifen oder vorbeilaufen oder die jeweilige Dichtklappe 4a, 4b passieren kann.

Im konkreten Ausführungsbeispiel gemäß Figur 1 sind die Dichtklappen 4a an den Außenkanten 4a im Bereich der radial äußeren Gehäusewand 2 des Pumpengehäuses 2 um und über beispielsweise einen durchgehenden oder zwei einzelne Stift 13 am radial äußeren, pumpengehäuseseitigem Ende der Dichtklappen 4a schwenkbar gelagert, so dass sie in den Freiraum 29 in der radial äußeren Gehäusewand 2 schwenken können. Die Stifte 13 sind beispielsweise in den das Pumpengehäuse 2 bzw. die Pumpenkammer 8 axial begrenzenden Gehäusedeckel 9, 10 geführt bzw. aufgenommen.

In der Ausführung gemäß den Figuren 2 bis 4 sind die Dichtklappen 4b als Omega-Klappen 4b ausgebildet. Die in der Kontur kreisbogenförmigen bzw. teilzylindermantelförmigen Omega-Klappen 4b sind jeweils mittels Speichen 33 an einen Drehpunkt 28 geführt und an diesem Drehpunkt 28 über jeweils einen Stift 13 in den Gehäusedeckel 9, 10 des Pumpengehäuses drehbar gelagert. Die kreisbogenförmigen bzw. teilzylindermantelförmigen Omega-Klappen 4b ragen einen Freiraum 29 bzw. eine Ausweitung 29 in dem dafür geweiteten Pumpengehäuse 2. Dabei weisen neben der Gehäusewand 2 auch die Gehäusedeckel 9, 10 des Pumpengehäuses 2 den Freiraum 29 bzw. die Ausweitung 29 insbesondere im Bewegungsbereich der Omega-Klappen 4b und der Speichen 33 auf. Der jeweilige Flügel 3 ist somit in der Lage an der jeweils sich öffnenden Omega-Klappe 4b entlangzustreifen bzw. vorbeizulaufen, während die jeweilige Omega-Klappe 4b sich, von der Bewegungsrichtung der Rotationsrichtung 14 der Flügel 3 her entgegengesetzt, um das Ende des jeweiligen Flügels 3 bewegt bzw. dreht, ohne dass es zu einer Kollision kommt.

Durch die Dichtklappen 4a, 4b werden Pumpenkammerabschnitte 8a gebildet, wobei in Rotationsrichtung 14 nach den Dichtklappen 4a, 4b die Sogseite 6 oder Zulaufseite 6 und vor den Dichtklappen 4a, 4b die Druckseite 7 oder Ablaufseite 7 ist.

Wie oben beschrieben wird die Rotationsgeschwindigkeit der Flügel 3 in Bereich bzw. Bewegungsbereich der Dichtklappen 4a, 4b deutlich verringert, wodurch die Synchronisation der jeweiligen Bewegungen vereinfacht und auch Kollisionen vermieden werden.

Durch den Freiraum 29 bzw. die Ausweitung 29 wird eine Verwirbelung des Fluids, insbesondere Blutes bei Öffnen der Dichtklappen 4a, 4b durch das Passieren des jeweiligen Flügels 3 erreicht.

Dies verstärkend ist es zudem vorgesehen, dass der Drehpunkt 28 in Bezug zu der Kontur kreisbogenförmigen bzw. teilzylindermantelförmigen Omega-Klappen 4b nicht zentrisch sondern exzentrisch angeordnet ist, wobei der Drehpunkt 28 so gewählt wird, dass mit Einsetzen der Drehbewegung oder Öffnungsbewegung der Dichtklappen 4b bzw. Omega-Klappen 4b diese sich von allen am Kreisbogen bzw. Teilzylindermantel bestehenden Kontaktstellen 38 oder Berührungsstellen 38 und damit Abdichtungsstellen 38 zum Pumpengehäuse 2 und insbesondere zur Gehäusewand 2, den Gehäusedeckeln 9, 10 und deren Angrenzungsbereiche löst und damit bereits die Verwirbelung und Umspülung sowohl an den Klappenrändern wie deren Innen- wie Außenflächen insbesondere durch die Freibereiche 29 bzw. der Ausweitung 29 wie des Freiraumes 37 zwischen der Omega-Klappe 4b und dem Flügel 3 freigegeben einsetzt.

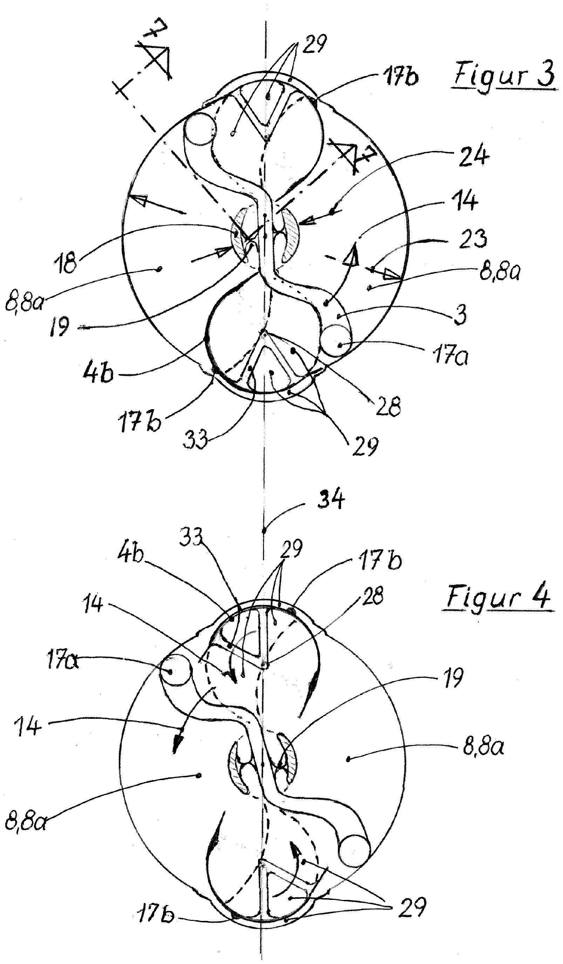

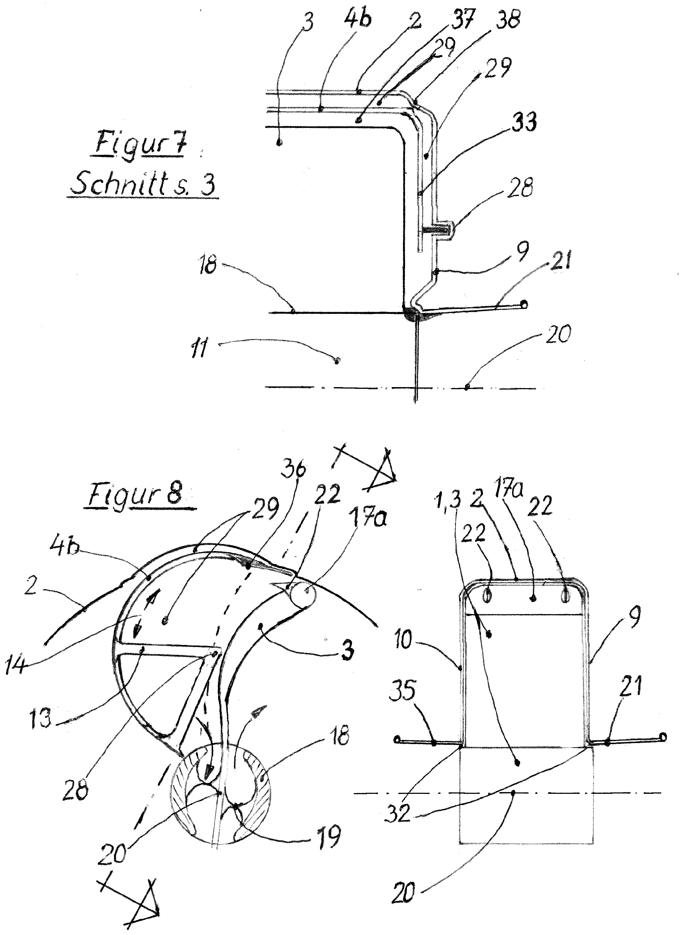

In Figur 7 ist als Schnitt eines Details zur Figur 3 ein Teil des Pumpengehäuses 2 mit Gehäusewand 2 und einem Gehäusedeckel 9, der Dichtklappe 4b als Omega-Klappe 4b mit Speichen 33, die zu einem Drehpunkt 28 geführt sind, das Rotorachsrohr 18 mit Rotor 1 und Flügel 3 sowie der Rotorachse 20 als Symmetrieachse und einem Eingangsrohr 21 zum Gehäusedeckel 9 und Rotorachsrohr 18 mit dem Einlaufkanal 11 bzw. den Einlauf 11 zum Rotorachsrohr 18 dargestellt. Im Bereich der Omega-Klappe 4b sowie im Bereich der Speichen 33 ist im Pumpengehäuse 2 ein Freiraum 29 bzw. eine Ausweitung 29 vorhanden. Im Eckbereich bzw. im Übergang von der Gehäusewand 2 und zum Gehäusedeckel 9 wird bei geschlossener Dichtklappe 4b als Omega-Klappe 4b eine Kontaktstelle 38 oder Berührungsstelle 38 und damit Abdichtungsstelle 38 zwischen Dichtklappe 4b als Omega-Klappe 4b und dem Pumpengehäuse 2, insbesondere zum jeweiligen Gehäusedeckel 9 gebildet.

Weiterhin ist im Bereich der jeweiligen Dichtklappe 4b bzw. Omega-Klappe 4b radial außen wie jeweils axial ein Abstand 37 zwischen der jeweiligen Dichtklappe 4b bzw. Omega-Klappe 4b und dem passierenden Flügel 3 vorhanden. Wie dargestellt, entspricht der Abstand 37 annähernd dem Freiraum 29 bzw. der Ausweitung 29 zwischen der jeweiligen Dichtklappe 4b bzw. Omega-Klappe 4b und der im Bereich der jeweiligen Dichtklappe 4b bzw. Omega-Klappe 4b geweiteten radialen Gehäusewand 2 bzw. dem in diesem Bereich geweiteten axialen Gehäusedeckel 9.

Um den Flügel, wie in den Figuren 2 bis 4 dargestellt, möglichst nah an der Kontur der kreisbogenförmigen bzw. teilzylindermantelförmigen Omega-Klappen 4b entlangstreifen bzw. vorbeilaufen zu können, weisen die Flügel 3 in radialer Richtung einen s-förmigen und damit mehrfach gekrümmten Verlauf auf. Vorteilhaft sind der in radialer Richtung gekrümmte Verlauf der Kontur der Flügel 3 und der gekrümmte Verlauf der Kontur der zumindest zwei Dichtklappen 4a, 4b zueinander ähnlich oder angepasst, so dass vor dem Öffnen der Dichtklappe möglichst viel des Fluides insbesondere das Blut noch gefördert werden kann. Dies schließt die Sogseite 6 oder Zulaufseite 6 und Druckseite 7 oder Ablaufseite 7 gleichermaßen ein, da der Sog 6 und der Druck 7 erfolgen, so lange die Dichtklappe 4b, als Omega-Klappe 4b noch geschlossen ist.

Im konkreten Ausführungsbeispiel der Figur 1 sind die Flügel 3 radial geradlinig ausgebildet, so dass diese problemlos an den Dichtklappen 4a entlangstreifen oder vorbeilaufen können, wobei in dieser Klappenlösung ein großer Drehwinkelbereich ohne Fördern eintritt, da die Dichtklappe 4a schon mit Beginn des einlaufenden Flügels 3 vollkommen geöffnet sein muss.

Der Antrieb der Flügel 3 bzw. des Rotors 1 ist, wie in Figur 1 bis 4 dargestellt, mittels Magneten 17a an den radialen Enden der Flügel 3 und, wie in der Figur 2 dargestellt, mittels Elektromagneten 16a an oder im Bereich der radialen Gehäusewand 2 des Pumpengehäuses 2 realisiert. Die Elektromagnete 16a, 16b lassen sich einzeln und/oder als Gruppe ansteuern, so dass je nach Winkelstellung der Flügel 3 wie oben dargestellt unterschiedliche Rotationsgeschwindigkeiten eingestellt werden können.

Ebenso sind an den Dichtklappen 4b bzw. Omega-Klappen 4b auch Magneten 17b vorgesehen, mit welchen die Stellung der Dichtklappen 4b bzw. Omega-Klappen 4b veränderbar und im Bedarfsfalle haltbar bzw. feststellbar ist. Dementsprechend sind am Pumpengehäuse 2 bzw. an den Gehäusedeckeln 9, 10 Elektromagneten 16b vorgesehen, die entsprechend ansteuerbar sind, siehe beispielhaft in Figur 2.

In den Figuren 2 bis 4 sind unterschiedliche Betriebszustände bzw. Betriebsstellungen der Drehkolbenpumpe bzw. Drehflügelpumpe dargestellt. Während in Figur 2 die Dichtklappen 4b geschlossen sind und die Flügel 3 mit einer hohen Rotationsgeschwindigkeit sowohl für Sog 6 wie für Druck 7 sorgen sind bei der Figur 3 die Dichtklappen 4b nach dem Passieren der Flügel 3 noch geöffnet. Die Geschwindigkeit ist noch gering und kann jedoch, nachdem die Dichtklappen 4b vollständig geschlossen sind wieder erhöht werden. In Figur 4 ist dargestellt, dass die Dichtklappen 4b sich wieder schließen und dabei der sogseitigen Kontur der Flügel 3 folgen können, um sich nach den geringfügig weitergedrehten Flügeln 3 alsbald zurückdrehend zu schließen, so dass der neue Pumpvorgang beginnen kann.

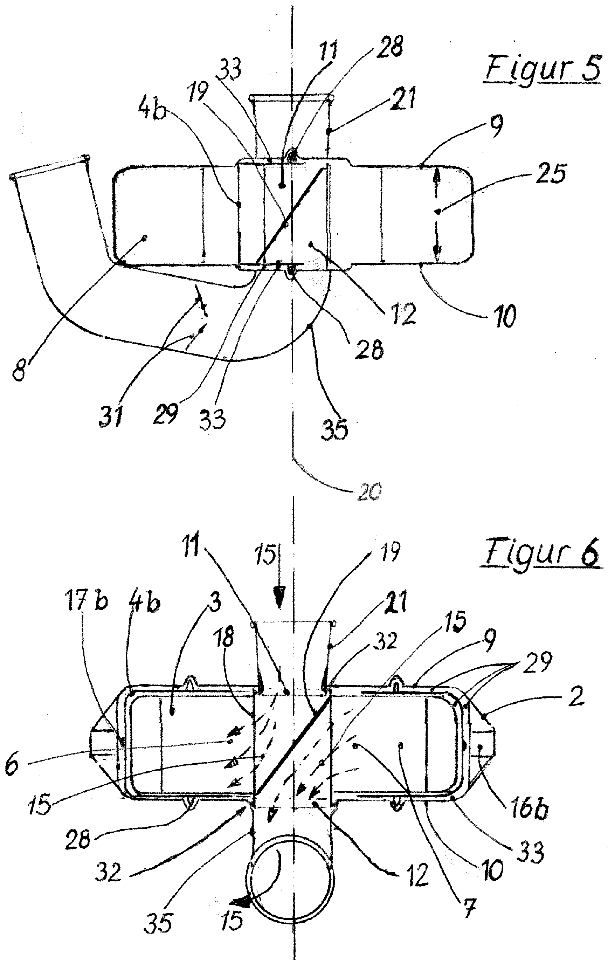

Die Figur 5 zeigt eine Schnittdarstellung der Figur 2, wobei im Bereich der Omega-Klappe 4b sowie im Bereich der Speichen 33 in den Gehäusedeckeln 9, 10 des Pumpengehäuses 2 ein Freiraum 29 bzw. eine Ausweitung 29 vorhanden ist, in der die Dichtklappe 4b als Omega-Klappe 4b mit Speichen 33, die zu einem Drehpunkt 28 geführt sind, beweglich angeordnet ist. In der Figur oben ist als Eingangsseite das Eingangsrohr 21 im oder am Gehäusedeckel 9 dargestellt, an welches der Einlaufkanal 11 im Rotorachsrohr 18 daran die Einlassöffnung nicht dargestellt in die Pumpenkammer 8 mit einer Pumpenkammertiefe 25, was der Rotorachsrohrlänge entspricht, anschließt. Auf der Ausgangseite ist im Rotorachsrohr 18 die Auslassöffnung nicht dargestellt, an welche sich der Auslaufkanal 12 im Rotorachsrohr 18 und daran das unten dargestellte Ausgangsrohr 35 im oder am Gehäusedeckel 10 anschließt. Im Ausgangsrohr 35 sind zudem Segelklappen 31 vorgesehen. Im Rotorachsrohr 18 ist eine Kanaltrennung 19 vorhanden, die schräg verlaufend den Einlaufkanal 11 vom Auslaufkanal 12 axial trennt.

In Figur 6 ist als Schnittdarstellung eine Drehkolbenpumpe bzw. Drehflügelpumpe am Ende des Förderns, kurz vor dem Öffnen der Dichtklappen 4b dargestellt. Während die Flügel 3 noch in Richtung der Dichtklappen 4b beispielsweise als Omega-Klappen 4b, welche Speichen 33 aufweisen, die zum Drehpunkt 28 geführt sind, drehen, strömt mit Fließrichtung 15 in die Pumpe und Pumpenkammer 8 auf der Sogseite 6 der Flügel 3 und der Dichtklappe 4b noch Fluid oder Blut über das Eingangsrohr 21, den Einlaufkanal 11 und die Einlassöffnung 6 in die Pumpenkammer 8, während auf der Druckseite 7 der Flügel 3 und der Dichtklappe 4b mit Fließrichtung 15 aus der Pumpe noch Fluid oder Blut aus der Pumpenkammer 8 über die Auslassöffnung 7, den Auslaufkanal 12 und das Ausgangsrohr 35 strömt. Über die Elektromagneten 16b werden die Flügel 3 mit ihren Magneten nicht dargestellt entsprechend angesteuert und vor den sich zu öffnenden Dichtklappen 4b zunächst abgebremst. Die Dichtklappen 4b mit ihren Magneten 17b werden über die Elektromagneten 16b angesteuert, so dass die Dichtklappen 4b sich rechtzeitig öffnen und die Flügel 3 sich langsam und synchron zu den Dichtklappen 4b unter diesen durchdrehen. Der Rotor 1 mit den Flügeln 3 weist eine Rotorlagerung 32 in den das Pumpengehäuse 2 axial begrenzenden Gehäusedeckeln 9, 10 an den Übergangsstellen zwischen Eingangsrohr 21 und Rotorachsrohr 18 sowie zwischen Rotorachsrohr 18 und Ausgangsrohr 35 auf. Im Rotorachsrohr 18 ist ebenfalls die Kanaltrennung 19 vorhanden.

In Figur 8 ist ein weitere Ausführung eines Flügels 3 dargestellt, welcher entgegen der Rotationsrichtung 14 einen sichelförmig gekrümmten Verlauf der Kontur aufweist und an den radialen Enden in Rotationsrichtung Überstände 22 in Form einer Nase oder mehr Nasen vorhanden sind, die in der Lage sind, eine eventuell nicht vollständig an der radial äußeren Gehäusewand 2 anliegende Omega-Klappe 4b für ein freies Passieren des Flügels 3 an die radial äußeren Gehäusewand 2 zu drücken.

Weiterhin ist an der Dichtklappe 4b als Omega-Klappe 4b die annähernd tangentiale Verlängerung 36 mit dem Eintritts- bzw. Austrittsradius 23 entsprechend des Pumpenkammeraußendurchmessers 23 der Gehäusewand 2 der Pumpenkammer 8 bzw. mit dem Pumpenkammeraußendurchmesser 23 vorhanden.

Der Ausschnitt zeigt weiterhin ein Teil des Pumpengehäuses 2 mit Gehäusewand 2 und die Dichtklappe 4b als Omega-Klappe 4b mit Speichen 33, die zu einem Drehpunkt 28 geführt sind. Im Bereich der Omega-Klappe 4b sowie im Bereich der Speichen 33 ist im Pumpengehäuse 2 ein Freiraum 29 bzw. eine Ausweitung 29 vorhanden. Im Rotorachsrohr 18 ist ebenfalls die Kanaltrennung 19 vorhanden. Der Rotor 1 mit den Flügeln 3 weist eine Rotorlagerung 32 in den das Pumpengehäuse 2 axial begrenzenden Gehäusedeckeln 9, 10 an den Übergangsstellen zwischen Eingangsrohr 21 und Rotorachsrohr 18 sowie zwischen Rotorachsrohr 18 und Ausgangsrohr 35 auf, wie es in der Seitenansicht in Figur 8 dargestellt ist.

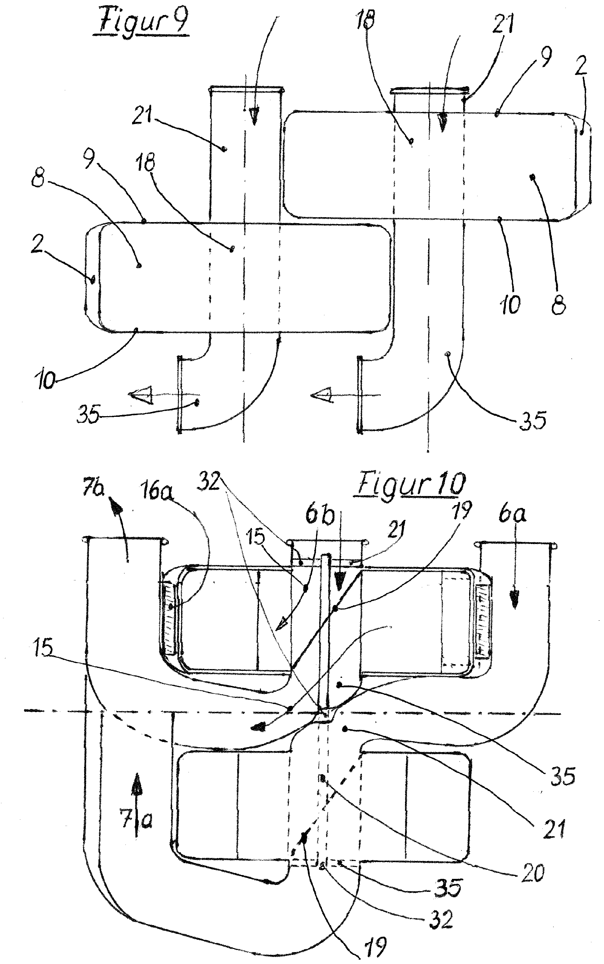

Die Figuren 9 und 10 zeigen eine jeweils kombinierte Anordnung zweier Drehkolbenpumpen bzw. Drehflügelpumpen. In Figur 9 weist jede der Pumpen einen eigenen Antrieb für den Rotor 1 bzw. die Flügel 3 auf. In Figur 10 ist ein für beide Pumpen gemeinsamer Antrieb in der oben dargestellten Pumpe vorhanden, welcher wie oben beschrieben aufgebaut ist, wobei der Antrieb des zweiten Rotors 1 über die Rotorachse 20, welche damit eine Welle ist, erfolgt und die zugleich Bestandteil des Rotors 1 ist. Die Rotorlagerung 32 der Rotorachse 20 ist im Eingangsrohr 21 der oben dargestellten Pumpe und im Ausgangsrohr 35 der unten dargestellten Pumpe sowie zwischen den Pumpen im Übergang zwischen dem Ausgangsrohr 35 der oben dargestellten Pumpe und dem der Eingangsrohr 21 der unten dargestellten Pumpe vorhanden.

Für die Anordnung lassen sich die beiden Rotorachsen 20 als Wellen zusammenstecken. Mittels dieser Anordnung kann beispielsweise ein Herzvollersatz realisiert werden, da beide Kammern synchron, jedoch strömungsseitig getrennt arbeiten, wobei Blut aus Richtung der Mitralklappe 6a durch die untere Pumpe gefördert zur Aorta 7a gelangt, während Blut aus Richtung der Trikuspidalklappe 6b durch die obere Pumpe zur Lungenarterie 7b gelangt.

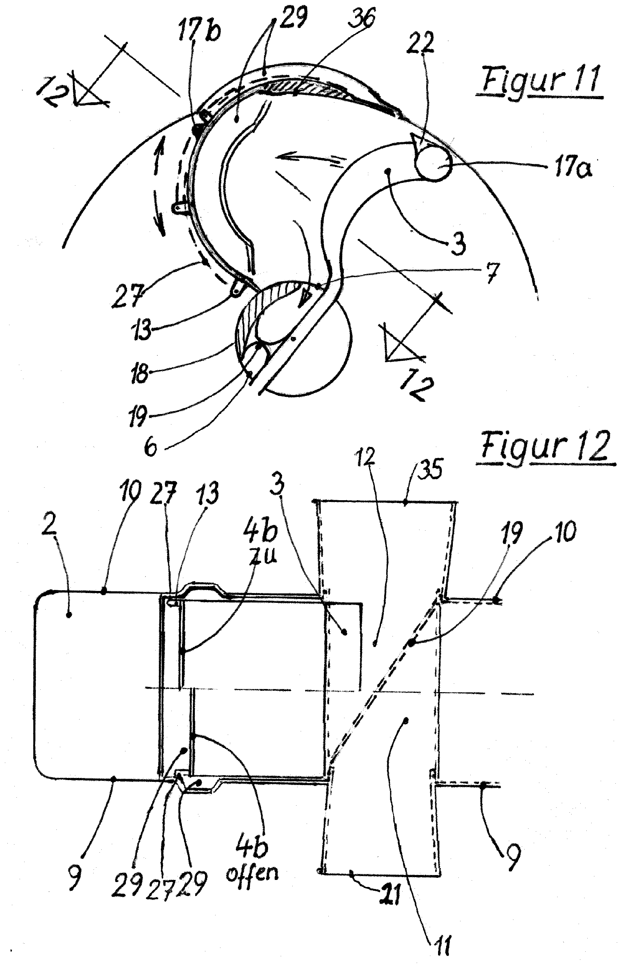

Die Figuren 11 und 12 zeigen eine weitere Ausführung der Lagerung und Führung der Dichtklappe 4b bzw. Omega-Klappe 4b.

Zur Führung der Dichtklappe 4b sind, wie in Figur 11 dargestellt, in den Gehäusedeckeln 9, 10 Führungsrillen 27 passend zur kreisbogenförmigen bzw. teilzylindermantelförmigen Kontur der Dichtklappe 4b, wobei die Führungsrillen 27 so angeordnet sind, dass sich eine exzentrische Lagerung ergibt und bei einer Drehbewegung die Dichtklappen 4b sich zumindest von der radial äußeren Gehäusewand 2 bzw. dem Anlagebogen der Dichtklappe 4b mit dem Pumpengehäuse 2 wegbewegen. Beispielsweise sind zwei oder drei Führungsrillen 27 vorhanden, die nicht konzentrisch zueinander sind und zudem jeweils einen sich verengenden Kurvenverlauf aufweisen.

Die Dichtklappen weisen Stege 13 die Stifte 13 oder Kugeln 13 umfassen, welche in den Führunsgsrillen ragen bzw. gleiten oder rollen können und somit die Bewegung der Dichtklappen 4b und deren leichtgängige Führung realisieren.

Wie in Figur 12 dargestellt ist, ist auf der Druckseite 7 der Dichtklappe 4b in den Gehäusedeckeln 9, 10 jeweils entlang des Verlaufes der geschlossenen Dichtklappe 4b die Ausweitung 29 in Form einer Rinne in den Gehäusedeckeln 9, 10 kreisbogenartig nur kurz vor der Dichtungsanlage zwischen Pumpengehäuse 2 und Dichtklappe 4b vorhanden. Die Dichtklappe 4b dichtet im geschlossenen Zustand am Rotorachsrohr 18 und an den Gehäusedeckeln 9, 10 abseits der Ausweitung 29 sowie an der radial äußeren Gehäusewand 2 ab, wie es in der Figur 12 in der oberen Hälfte dargestellt ist. Beim Öffnen der Dichtklappe 4b und durch den nicht zueinander konzentrischen und zudem jeweils sich verengenden Kurvenverlauf der Führungsrillen 27 in Richtung der Ausweitung 29 bewegt sich die Dichtklappe 4b nicht nur vom Rotorachsrohr 18 weg, sondern auch in Richtung der bzw. über die Ausweitung 29 und auch weg von der anliegenden Stellung an der radial äußeren Gehäusewand 2, wie es in Figur 12 in der unteren Hälfte dargestellt ist. Somit wird ein Umspülen und Verwirbeln der Bereiche der Ausweitung 29 begünstigt. Für die weiteren Elemente kann auf die vorhergehenden Ausführungen verwiesen werden.

### Zusammenstellung der Bezugszeichen

1 - Rotor, Drehflügelrotor
2 - Pumpengehäuse, Gehäusewand
3 - Flügel
4a - Dichtklappe
4b - Dichtklappe, Omega-Klappe
6 - Sog, Sogseite, Zulauf, Zulaufseite , Einlassöffnung
6a - aus Richtung der Mitralklappe
6b - aus Richtung der Trikuspidalklappe
7 - Druck, Druckseite, Ablauf, Ablaufseite, Auslassöffnung
7a - zu Aorta
7b - zu Lungenarterie
8 - Pumpenkammer
8a - Pumpenkammerabschnitt
9 - Gehäusedeckel, Eingang
10 - Gehäusedeckel, Ausgang
11 - Einlaufkanal, Einlauf Rotorachsrohr
12 - Auslaufkanal, Auslauf Rotorachsrohr
13 - Nocke, Steg, Klappenführung, Stift, Kugel
14 - Rotationsrichtung
15 - Fließrichtung
16a - Elektromagneten Rotorbewegung
16b - Elektromagnet Klappenarretierung
17a - Magnet an Flügelaußenkante
17b - Magnet an Dichtklappe, Magnet an Omega-Klappe
18 - Rotorachsrohr
19 - Kanaltrennung
20 - Rotorachse
21 - Eingangsrohr
22 - Überstand
23 - Pumpenkammeraußendurchmesser, Eintrittsradius, Austrittsradius
24 - Pumpenkammerinnendurchmesser, Rotorachsrohrdurchmesser
25 - Pumpenkammertiefe
26 - Bypass, Ventil
27 - Führungsrille, Führungen, Schiene,
28 - Drehpunkt, Schwenkpunkt
29 - Freiraum, Ausweitung
30 - Zylinderwandung
31 - Segelklappe
32 - Rotorlagerung
33 - Speiche
34 - Konstruktionsachse
35 - Ausgangsrohr
36 - Verlängerung
37 - Abstand
38 - Kontaktstelle, Berührungsstelle, Abdichtungsstelle

## Patentansprüche

1. Drehkolbenpumpe, umfassend ein Pumpengehäuse (2, 9, 10) mit einer im Wesentlichen zylindrischen Pumpenkammer (8) und einen Drehkolben als Rotor (1) mit zumindest zwei in Umfangsrichtung gegenüberliegend oder gleichmäßig verteilt angeordneten Flügeln (3) und zumindest einer Dichtklappe (4),
**dadurch gekennzeichnet,**
**dass** zumindest zwei in Umfangsrichtung gegenüberliegend oder gleichmäßig verteilt angeordnete Dichtklappen (4a, 4b) vorhanden sind, wobei die zumindest zwei Dichtklappen (4a, 4b) drehbar oder schwenkbar sind, und wobei in einem Rotorachsrohr (18) sich jeweils von den gegenüberliegenden axialen Enden erstreckend und voneinander getrennt axial ein Einlaufkanal (11) zu zumindest zwei Einlassöffnungen (6) in die Pumpenkammer (8) und ein Auslaufkanal (12) von zu zumindest zwei Auslassöffnungen (7) aus der Pumpenkammer (8) vorhanden ist, wobei jeweils eine der Einlassöffnungen (6) in Rotationsrichtung (14) nach und jeweils eine der Auslassöffnungen (7) in Rotationsrichtung (14) vor jeweils einem der Flügel (3) im Rotorachsrohr (18) angeordnet ist.

2. Drehkolbenpumpe nach Anspruch 1,
**dadurch gekennzeichnet,**
**dass** die Flügel (3) punktsymmetrisch zur Rotationsachse des Rotorachsrohrs (18) sind und/oder dass die Kontur der Flügel (3) in radialer Richtung einen einfach oder mehrfach gekrümmten Verlauf aufweist und/oder dass die Kontur der zumindest zwei Dichtklappen (4a, 4b) in radialer Richtung einen gekrümmten oder kreisbogenförmigen Verlauf aufweist und/oder dass der in radialer Richtung gekrümmte Verlauf der Kontur der Flügel (3) und der gekrümmte Verlauf der Kontur der zumindest zwei Dichtklappen (4a, 4b) zueinander ähnlich oder angepasst oder kongruent sind.

3. Drehkolbenpumpe nach einem der vorhergehenden Ansprüche,
**dadurch gekennzeichnet,**
**dass** die zumindest zwei Dichtklappen (4a, 4b) an zumindest dem jeweils zur radial äußeren Gehäusewand (2) der Pumpenkammer (8) weisenden Ende eine annähernd tangential geradlinige Verlängerung (36) oder eine Verlängerung (36) mit einem Eintritts- bzw. Austrittsradius (23) entsprechend des Pumpenkammeraußendurchmessers (23) der Gehäusewand (2) der Pumpenkammer (8) oder einem Pumpenkammeraußendurchmesser (24) aufweisen.

4. Drehkolbenpumpe nach einem der vorhergehenden Ansprüche,
**dadurch gekennzeichnet,**
**dass** die zumindest zwei Dichtklappen (4a, 4b) im oder am Pumpengehäuse (2) über Speichen (33) oder Stege (35) oder Nocken (13) oder Stifte oder Kugeln an einem Drehpunkt (28) oder Schwenkpunkt oder in Schienen (27) oder in oder auf Führungen (27) gleitbar oder drehbar oder schwenkbar gelagert oder angeordnet sind, wobei das Pumpengehäuse (2) im Bereich oder Bewegungsbereich der zumindest zwei Dichtklappen (4a, 4b) zumindest bereichsweise radial und/oder axial zumindest einen Freiraum (29) aufweist.

5. Drehkolbenpumpe nach einem der vorhergehenden Ansprüche,
**dadurch gekennzeichnet,**
**dass** die zumindest zwei Dichtklappen (4a, 4b) konzentrisch oder exzentrisch drehbar oder schwenkbar oder verdrehbar sind.

6. Drehkolbenpumpe nach einem der vorhergehenden Ansprüche,
**dadurch gekennzeichnet,**
**dass** das Pumpengehäuse (2) zumindest im radial äußeren Bereich über den Umfang und/oder über die axiale Erstreckung und/oder im Bereich der Dichtklappen (4) gleichmäßig und/oder unregelmäßig verteilt Elektromagnete (16a, 16b) aufweist.

7. Drehkolbenpumpe nach einem der vorhergehenden Ansprüche,
**dadurch gekennzeichnet,**
**dass** an den zumindest zwei Dichtklappen (4a, 4b) zumindest ein Magnet (17b) und/oder dass zumindest am radial äußeren Ende der Flügel zumindest ein Magnet (17a) vorhanden ist.

8. Drehkolbenpumpe nach einem der vorhergehenden Ansprüche,
**dadurch gekennzeichnet,**
**dass** die Elektromagnete (16a, 16b) einzeln und/oder als Gruppe ansteuerbar sind.

9. Drehkolbenpumpe nach einem der vorhergehenden Ansprüche,
**dadurch gekennzeichnet,**
**dass** die zumindest zwei Dichtklappen (4a, 4b) jeweils zumindest eine Dichtung und/oder ein Überströmventil (26) oder unterdruckgesteuerter Bypass (26) in der jeweiligen Dichtklappe (4a, 4b) oder am oder im Gehäuse (2) oder Rotor (1) zwischen Druck (7) oder Ablauf (7) und Sog (6) oder Zulauf (6) aufweisen.

10. Drehkolbenpumpe nach einem der vorhergehenden Ansprüche,
**dadurch gekennzeichnet,**
**dass** die Flügel radial im Bereich des Pumpengehäuses in Rotationsrichtung (14) einen Überstand (22) aufweisen und/oder dass die Flügel (3) und/oder dass Pumpengehäuse (2) im Bereich der Dichtklappen (4a, 4b) ein Überströmventil (26) oder unterdruckgesteuerter Bypass (26) zwischen Druck (7) oder Ablauf (7) und Sog (6) oder Zulauf (6)aufweisen.

11. Drehkolbenpumpe nach einem der vorhergehenden Ansprüche,
**dadurch gekennzeichnet,**
**dass** der Auslaufkanal (12) oder das Rotorachsrohr (12) für den Auslauf oder das Ausgangsrohr (35) einen gewunden oder schraubenförmigen Verlauf oder Innenquerschnitt aufweist.

12. Verfahren zum Betreiben einer Drehkolbenpumpe, vorzugsweise zum Betreiben einer Drehkolbenpumpe gemäß der Ansprüche 1 bis 13, insbesondere als Herzpumpe zur Unterstützung des menschlichen Herzens oder als Herzersatz, wobei in einer im Wesentlichen zylindrischen Pumpenkammer (8) eines Pumpengehäuses (2) durch die Drehbewegung eines Drehkolben als Rotor (1) mit einem Rotorachsrohr (18) und mit zumindest zwei in Umfangsrichtung gegenüberliegend oder gleichmäßig verteilt angeordneten Flügeln (3) ein Fluid in jeweils eine Auslassöffnung (7) in dem Rotorachsrohr (18) in Rotationsrichtung (14) vor dem jeweiligen Flügel (3) gepresst und zugleich das Fluid durch eine Einlassöffnung (6) in dem Rotorachsrohr (18) in Rotationsrichtung (14) nach dem jeweiligen Flügel (3) angesaugt wird, wobei eine Trennung der Bereiche des Drucks (7) oder Ablaufs (7) und des Sogs (6) oder Zulaufs (6) jeweils mittels einer Dichtklappe (4a, 4b) erfolgt und die jeweilige Dichtklappe (4a, 4b) sich für den Druck (7) oder Ablauf (7) und den Sog (6) oder Zulauf (6) in einer geschlossenen Stellung befindet und nach dem Druck (7) oder Ablauf (7) sowie nach dem Sog (6) oder Zulauf (6) für das Passieren des jeweiligen Flügels (3) die jeweilige Dichtklappe (4a, 4b) an den radial äußeren Bereich der Pumpenkammer (8) geschwenkt oder um das radial äußeren Ende des jeweiligen Flügels (3) gedreht wird und nach dem Passieren die jeweilige Dichtklappe (4a, 4b) wieder in eine geschlossene Stellung bewegt wird und danach der Druck (7) oder Ablauf (7) und der Sog (6) oder Zulauf (6) erneut erfolgen.

13. Verfahren nach Anspruch 12,
**dadurch gekennzeichnet,**
**dass** die Drehbewegung des Drehkolbens als Rotor (1) mit je nach Winkelstellung der Flügel (3) unterschiedlicher Rotationsgeschwindigkeit erfolgt und/oder dass die Drehbewegung des Drehkolbens als Rotor (1) verringert wird, wenn sich der jeweilige Flügel (3) im Bereich der jeweiligen Dichtklappe (4a, 4b) befindet.

14. Verfahren nach einem der Ansprüche 12 und 13,
**dadurch gekennzeichnet,**
**dass** die Einstellung der Drehbewegung des Drehkolbens als Rotor (1) und/oder Stellung der Dichtklappen (4a, 4b) mittels der Ansteuerung von Elektromagneten (16a, 16b) erfolgt und/oder dass die Drehbewegung des Rotors (1), die Stellung der Dichtklappen (4a, 4b) und/oder weitere Daten als Basis für den Betrieb der Pumpe erfassen werden und der Betrieb der Pumpe gesteuert wird.

15. Verwendung der Drehkolbenpumpe nach einem der Ansprüche 1 bis 11 als Herzpumpe zur Unterstützung des menschlichen Herzens oder als Herzersatz.

## Claims

1. Rotary piston pump, comprising a pump casing (2, 9, 10) with a substantially cylindrical pump chamber (8) and a rotary piston as a rotor (1) with at least two blades (3) arranged opposite each other in the circumferential direction or distributed evenly, and at least one sealing flap (4a, 4b),
**characterised in**
**that** at least two sealing flaps (4a, 4b) are arranged opposite each other or distributed evenly in the circumferential direction, wherein the at least two sealing flaps (4a, 4b) are rotatable or pivotable, and wherein an inlet channel (11) extends axially from the respective opposite axial ends in a rotor axle tube (18) and is separate from an axial outlet channel (12), which leads to at least two inlet openings (6) into the pump chamber (8) and at least two outlet openings (7) from the pump chamber (8), wherein each of the inlet openings (6) is arranged in the direction of rotation (14) behind and each of the outlet openings (7) in the direction of rotation (14) ahead of one of the blades (3) in the rotor axle tube (18).

2. Rotary piston pump according to claim 1,
**characterised in**
**that** the blades (3) are point-symmetric to the axis of rotation of the rotor axle tube (18) and/or the contour of the blades (3) has a simple or multiple curvature in the radial direction, and/or the contour of the at least two sealing flaps (4a, 4b) has a curved or circular arc shape in the radial direction, and/or the radial curvature of the blade contour (3) and the curved contour of the at least two sealing flaps (4a, 4b) are similar, adapted, or congruent to each other.

3. Rotary piston pump according to any of the preceding claims,
**characterised in**
**that** the at least two sealing flaps (4a, 4b) have an approximately tangential linear extension (36) or an extension (36) with an entry or exit radius (23) corresponding to the outer diameter of the pump chamber (23) of the casing wall (2) or an outer diameter of the pump chamber (24) at least at the end facing the radially outer casing wall (2) of the pump chamber (8).

4. Rotary piston pump according to any of the preceding claims,
**characterised in**
**that** the at least two sealing flaps (4a, 4b) are mounted or arranged in or on the pump casing (2) via spokes (33) or rod (35) or cams (13) or pins or balls at a pivot point (28) or swivel point or in tracks (27) or in or on guides (27), such that they are slidable, rotatable, or pivotable, wherein the pump casing (2) has at least in the area or movement range of the at least two sealing flaps (4a, 4b) at least partially a radial and/or axial clearance (29).

5. Rotary piston pump according to any of the preceding claims,
**characterised in**
**that** the at least two sealing flaps (4a, 4b) are concentrically or eccentrically rotatable, pivotable, or twistable.

6. Rotary piston pump according to any of the preceding claims,
**characterised in**
**that** the pump casing (2) has electromagnets (16a, 16b) evenly or irregularly distributed over the circumference and/or the axial extension and/or in the area of the sealing flaps (4).

7. Rotary piston pump according to any of the preceding claims,
**characterised in**
**that** at least one magnet (17b) is present on the at least two sealing flaps (4a, 4b) and/or at least one magnet (17a) is present at the radially outer end of the blades.

8. Rotary piston pump according to any of the preceding claims,
**characterised in**
**that** the electromagnets (16a, 16b) can be controlled individually and/or as a group.

9. Rotary piston pump according to any of the preceding claims,
**characterised in**
**that** the at least two sealing flaps (4a, 4b) each have at least one seal and/or an overflow valve (26) or a vacuum-controlled bypass (26) in the respective sealing flap (4a, 4b) or on or in the casing (2) or rotor (1) between the pressure (7) or outlet (7) and suction (6) or inlet (6).

10. Rotary piston pump according to any of the preceding claims,
**characterised in**
**that** the blades (3) radially project in the area of the pump casing in the direction of rotation (14) and/or that the blades (3) and/or the pump casing (2) have an overflow valve (26) or a vacuum-controlled bypass (26) between the pressure (7) or outlet (7) and the suction (6) or inlet (6) in the area of the sealing flaps (4a, 4b).

11. Rotary piston pump according to any of the preceding claims,
**characterised in**
**that** the outlet channel (12) or the rotor axle tube (12) for the outlet or the output tube (35) has a winding or helical shape or cross-section.

12. Method for operating a rotary piston pump, preferably for operating a Rotary Piston Pump according to claims 1 to 13, wherein in a substantially cylindrical pump chamber (8) of a pump casing (2), a fluid is pressed through an outlet opening (7) in the rotor axle tube (18) in the direction of rotation (14) ahead of the respective blade (3) by the rotational movement of a rotary piston as a rotor (1) with a rotor axle tube (18) and with at least two blades (3) arranged opposite each other in the circumferential direction or evenly distributed, and simultaneously, the fluid is sucked through an inlet opening (6) in the rotor axle tube (18) in the direction of rotation (14) behind the respective blade (3), wherein a separation of the pressure (7) or outlet (7) and suction (6) or inlet (6) areas is achieved by means of a sealing flap (4a, 4b), and the respective sealing flap (4a, 4b) remains in a closed position for the pressure (7) or outlet (7) and the suction (6) or inlet (6), and the respective sealing flap (4a, 4b) is pivoted towards the radially outer area of the pump chamber (8) or rotated around the radially outer end of the respective blade (3) to allow the passage of the blade (3), and after passage, the respective sealing flap (4a, 4b) is returned to a closed position, with the pressure (7) or outlet (7) and suction (6) or inlet (6) resuming thereafter.

13. Method according to claim 12,
**characterised in**
**that** the rotary movement of the rotary piston as a rotor (1) occurs at a varying rotational speed depending on the angular position of the blades (3) and/or that the rotary movement of the rotary piston as a rotor (1) is slowed when the respective blade (3) is in the area of the respective sealing flap (4a, 4b).

14. Method according to one of the claims 12 and 13,
**characterised in**
**that** the adjustment of the rotary movement of the rotary piston as a rotor (1) and/or the positioning of the sealing flaps (4a, 4b) is controlled via the actuation of electromagnets (16a, 16b), and/or that the rotary movement of the rotor (1), the positioning of the sealing flaps (4a, 4b), and/or additional data are collected as a basis for pump operation, with the pump's operation being regulated accordingly.

15. Use of the rotary piston pump according to any of claims 1 to 11 as a heart pump to assist the human heart or as a heart replacement.

## Revendications

1. Pompe à piston rotatif, comprenant un boîtier de pompe (2, 9, 10) avec une chambre de pompe (8) essentiellement cylindrique et un piston rotatif en tant que rotor (1) avec au moins deux palettes (3) disposées l'une en face de l'autre dans la direction périphérique ou réparties uniformément et au moins une plaque d'étanchéité (4a, 4b),
**caractérisée en ce que**
il y a au moins deux plaques d'étanchéité (4a, 4b) disposées l'une en face de l'autre dans la direction périphérique ou réparties uniformément, les deux plaques d'étanchéité (4a, 4b) minimales étant rotatives ou pivotantes, et un canal d'entrée (11) vers au moins deux ouvertures d'entrée (6) dans la chambre de pompe (8) et un canal de sortie (12) depuis au moins deux ouvertures d'entrée (7) hors de la chambre de pompe (8) étant présentes dans un tube d'axe de rotor (18) s'étendant respectivement depuis les extrémités axiales opposées et séparés axialement les uns des autres, l'une des ouvertures d'entrée (6) étant disposée dans le sens de rotation (14) après et l'une des ouvertures de sortie (7) étant disposée dans le sens de rotation (14) avant l'une des palettes (3) dans le tube d'axe de rotor (18).

2. Pompe à piston rotatif selon la revendication 1,
**caractérisée en ce que**
les palettes (3) sont à symétrie ponctuelle par rapport à l'axe de rotation du tube d'axe de rotor (18) et/ou **en ce que** le contour des palettes (3) présente un tracé à une ou plusieurs courbures dans la direction radiale et/ou **en ce que** le contour des deux plaques d'étanchéité (4a, 4b) minimales présente dans la direction radiale un tracé incurvé ou en arc de cercle et/ou que le tracé incurvé dans la direction radiale du contour des palettes (3) et le tracé incurvé du contour des deux plaques d'étanchéité (4a, 4b) minimales sont similaires ou adaptées ou congruentes entre elles.

3. Pompe à piston rotatif selon l'une des revendications précédentes,
**caractérisée en ce que**
les deux plaques d'étanchéité (4a, 4b) minimales présentent, au moins à l'extrémité tournée respectivement vers la paroi de boîtier (2) radialement extérieure de la chambre de pompe (8), un prolongement (36) rectiligne approximativement tangentiel ou un prolongement (36) avec un rayon d'entrée ou de sortie (23) correspondant au diamètre extérieur (23) de la paroi de boîtier (2) de la chambre de pompe (8) ou un diamètre extérieur (24) de la chambre de pompe.

4. Pompe à piston rotatif selon l'une des revendications précédentes,
**caractérisée en ce que**
les deux plaques d'étanchéité (4a, 4b) minimales sont montées ou disposées dans ou sur le boîtier de pompe (2) de manière à pouvoir glisser ou tourner ou pivoter sur un point de rotation (28) ou un point de pivotement ou dans des rails (27) ou dans ou sur des guides (27) par l'intermédiaire de rayons (33) ou d'entretoises (35) ou de cames (13) ou de goupilles ou de billes, le boîtier de pompe (2) présentant au moins un espace libre (29) dans la zone ou la zone de mouvement des deux plaques d'étanchéité (4a, 4b) minimales, au moins par zones, radialement et/ou axialement.

5. Pompe à piston rotatif selon l'une des revendications précédentes,
**caractérisée en ce que**
les deux plaques d'étanchéité (4a, 4b) minimales peuvent être tournées ou pivotées ou tournées de manière concentrique ou excentrique.

6. Pompe à piston rotatif selon l'une des revendications précédentes,
**caractérisée en ce que**
le boîtier de pompe (2) présente des électroaimants (16a, 16b) répartis de manière régulière et/ou irrégulière au moins dans la zone radialement extérieure sur la périphérie et/ou sur l'extension axiale et/ou dans la zone des plaques d'étanchéité (4).

7. Pompe à piston rotatif selon l'une des revendications précédentes,
**caractérisée en ce que**
au moins un aimant (17b) est présent sur les deux plaques d'étanchéité (4a, 4b) minimales et/ou **en ce qu'**au moins un aimant (17a) est présent au moins à l'extrémité radialement extérieure des palettes.

8. Pompe à piston rotatif selon l'une des revendications précédentes,
**caractérisée en ce que**
les électroaimants (16a, 16b) peuvent être commandés individuellement et/ou en groupe.

9. Pompe à piston rotatif selon l'une des revendications précédentes,
**caractérisée en ce que**
les deux plaques d'étanchéité (4a, 4b) présentent chacune au moins un joint d'étanchéité et/ou une soupape de décharge (26) ou une dérivation (26) commandée par dépression dans le clapet d'étanchéité respectif (4a, 4b) ou sur ou dans le corps (2) ou le rotor (1) entre la pression (7) ou l'évacuation (7) et l'aspiration (6) ou l'alimentation (6).

10. Pompe à piston rotatif selon l'une des revendications précédentes,
**caractérisée en ce que**
les palettes présentent radialement dans la zone du corps de pompe dans le sens de rotation (14) une saillie (22) et/ou **en ce que** les palettes (3) et/ou le boîtier de pompe (2) présentent dans la zone des plaques d'étanchéité (4a, 4b) une soupape de décharge (26) ou une dérivation commandée par dépression (26) entre la pression (7) ou l'écoulement (7) et l'aspiration (6) ou l'arrivée (6).

11. Pompe à piston rotatif selon l'une des revendications précédentes,
**caractérisée en ce que**
le canal de sortie (12) ou le tube d'axe de rotor (12) pour la sortie ou le tube de sortie (35) présente un tracé ou une section transversale intérieure enroulé ou hélicoïdal.

12. Procédé pour faire fonctionner une pompe à piston rotatif, de préférence pour faire fonctionner une pompe à piston rotatif selon les revendications 1 à 13, dans lequel, dans une chambre de pompe (8) sensiblement cylindrique d'un boîtier de pompe (2), par le mouvement de rotation d'un piston rotatif en tant que rotor (1) avec un tube d'axe de rotor (18) et avec au moins deux palettes (3) disposées en face l'une de l'autre dans la direction périphérique ou réparties de manière régulière, un fluide est introduit dans la chambre de pompe (8). fluide est pressé dans une ouverture de sortie (7) respective dans le tube d'axe de rotor (18) dans le sens de rotation (14) en amont de la pale respective (3) et, en même temps, le fluide est aspiré par une ouverture d'entrée (6) dans le tube d'axe de rotor (18) dans le sens de rotation (14) en aval de la pale respective (3), une séparation des zones de la pression (7) ou de l'évacuation (7) et de l'aspiration (6) ou de l'entrée (6) étant effectuée respectivement au moyen d'une plaque d'étanchéité (4a, 4b) et la plaque d'étanchéité respective (4a, 4b) se trouve dans une position fermée pour la pression (7) ou l'écoulement (7) et l'aspiration (6) ou l'arrivée (6) et, après la pression (7) ou l'écoulement (7) ainsi qu'après l'aspiration (6) ou l'arrivée (6) pour le passage de la pale (3) respective, la plaque d'étanchéité (4a, 4b) est pivotée vers la zone radialement extérieure de la chambre de pompe (8) ou est tournée autour de l'extrémité radialement extérieure de la pale respective (3) et, après le passage, la plaque d'étanchéité respective (4a, 4b) est à nouveau déplacée dans une position fermée et ensuite la pression (7) ou l'écoulement (7) et l'aspiration (6) ou l'arrivée (6) ont à nouveau lieu.

13. Procédé selon la revendication 12,
**caractérisée en ce que**
le mouvement de rotation du piston rotatif en tant que rotor (1) s'effectue avec une vitesse de rotation différente selon la position angulaire des palettes (3) et/ou **en ce que** le mouvement de rotation du piston rotatif en tant que rotor (1) est réduit lorsque la pale (3) respective se trouve dans la zone de la plaque d'étanchéité (4a, 4b) respective.

14. Procédé selon l'une des revendications 12 et 13,
**caractérisée en ce que**
le réglage du mouvement de rotation du piston rotatif en tant que rotor (1) et/ou de la position des plaques d'étanchéité (4a, 4b) s'effectue au moyen de la commande d'électroaimants (16a, 16b) et/ou **en ce que** le mouvement de rotation du rotor (1), la position des plaques d'étanchéité (4a, 4b) et/ou d'autres données sont détectées comme base pour le fonctionnement de la pompe et le fonctionnement de la pompe est commandé.

15. Utilisation de la pompe à piston rotatif selon l'une des revendications 1 à 11 comme pompe cardiaque pour assister le coeur humain ou comme substitut cardiaque.
